(19) Europäisches Patentamt
European Patent Office
Office européen des brevets

(11) Veröffentlichungsnummer: **0 112 803**
**B1**

(12) # EUROPÄISCHE PATENTSCHRIFT

(45) Veröffentlichungstag der Patentschrift: **02.09.87**

(21) Anmeldenummer: **83810604.5**

(22) Anmeldetag: **19.12.83**

(51) Int. Cl.⁴: **C 07 F 9/65**, C 07 F 9/40, C 07 F 9/38, C 07 F 9/32, C 07 F 9/30, C 07 F 9/53, A 01 N 57/24

(54) **Phosphorhaltige N-Phenylsulfonyl-N'-pyrimidinyl- und -triazinylharnstoffe.**

(30) Priorität: **27.12.82 CH 7562/82**

(43) Veröffentlichungstag der Anmeldung: **04.07.84 Patentblatt 84/27**

(45) Bekanntmachung des Hinweises auf die Patenterteilung: **02.09.87 Patentblatt 87/36**

(84) Benannte Vertragsstaaten: **AT BE CH DE FR GB IT LI NL SE**

(56) Entgegenhaltungen:
**GB-A-2 063 267**
**US-A-3 210 339**
**US-A-4 127 405**

**Journal OF MEDICINAL CHEMISTRY, Band 8, Nr. 3, Mai 1965, Seiten 377-383, Easton, Pennsylvania, US; F.A. WAGNER et al.: "Phosphorylated Benzenesulfonamides as animal systemic insecticides"**

(73) Patentinhaber: **CIBA- GEIGY AG, Klybeckstrasse 141, CH- 4002 Basel (CH)**

(72) Erfinder: **Maier, Ludwig, Dr., Im Lee 28, CH- 4144 Arlesheim (CH)**
Erfinder: **Meyer, Willy, Talweg 49, CH- 4125 Riehen (CH)**
Erfinder: **Oertle, Konrad, Dr., Vogesenstrasse 10, CH- 4106 Therwil (CH)**
Erfinder: **Roloff, Achim, Dr., Waldshuterstrasse 55, CH- 4310 Rheinfelden (CH)**
Erfinder: **Töpfl, Werner, Dr., Dornackstrasse 69, CH- 4143 Dornach (CH)**

EP 0 112 803 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue, herbizid wirksame und -pflanzenwuchsregulierende phosphorhaltige N-Phenylsulfonyl-N'pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmem des Pflanzenwachstums.

Die erfindungsgemässen phosphorphaltigen N-Phenylsulfonyl-N'-pyrimidinyl- und -triazinylharnstoffe haben die allgemeine Formel I

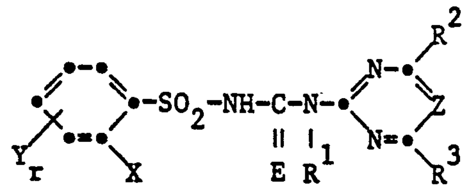

(I),

worin

X eine Gruppe

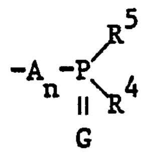

,

Y Wasserstoff, Halogen, $C_1$-$C_5$-Alkyl, Trifluormethyl, $C_2$-$C_5$-Alkenyl, $C_2$-$C_5$-Alkinyl, Nitro, -$COOR^6$ oder -Q-$R^6$,

Z Stickstoff oder die Methingruppe,

E Sauerstoff oder Schwefel,

$R^1$ Wasserstoff oder $C_1$-$C_5$-Alkyl und

$R^2$ und $R^3$ unabhängig voneinander $C_1$-$C_3$-Alkyl, $C_1$-$C_3$-Alkoxy, $C_1$-$C_3$-Halogenalkyl, $C_1$-$C_3$-Halogenalkoxy, Cyclopropyl, Amino, Methylamino oder Dimethylamino bedeuten, wobei

A für Sauerstoff, Schwefel, $C_1$-$C_5$-Alkylen, $C_2$-$C_5$-Alkenylen, $C_1$-$C_5$-Halogenalkylen, -$NR^7$-$(CH_2)_m$-; -$CH_2$-NH-$(CH_2)_m$ -$(CH_2)_m$ $NR^7$-, -O-$(CH_2)_p$-; -S-$(CH_2)_p$-, -$(CH_2)_p$-O- oder -$(CH_2)_p$-S-,

n für Null oder eins,

G für Sauerstoff oder Schwefel,

$R^4$ für $C_1$-$C_5$-Alkoxy, $C_1$-$C_5$-Halogenalkoxy, $C_1$-$C_5$-Alkylthio, $C_1$-$C_5$-Alkyl, $C_1$-$C_5$-Halogenalkyl, Phenyl oder Hydroxyl,

$R^5$ für Wasserstoff, $C_1$-$C_5$-Alkoxy, $C_1$-$C_5$-Halogenalkoxy, $C_1$-$C_5$-Alkylthio, $C_1$-$C_5$-Alkyl oder Hydroxyl,

$R^6$ für $C_1$-$C_5$-Alkyl, $C_1$-$C_5$-Halogenalkyl oder $C_2$-$C_6$-Alkoxyalkyl,

Q für Sauerstoff, Schwefel, -SO- oder -$SO_2$-,

$R^7$ für Wasserstoff, $C_1$-$C_5$-Alkyl, Phenyl, Benzyl oder durch $C_1$-$C_5$-Alkyl, Halogen oder Nitro substituiertes Phenyl,

m für eine Zahl von Null bis drei

p für eine Zahl von Null bis zwei und

r für eins oder zwei stehen.

Die Erfindung umfasst ausser den Verbindungen der Formel I auch deren Salze.

Herbizide und pflanzenwuchsregulierende Wirkstoffe aus der Klasse der Sulfonylharnstoffverbindungen sind seit einiger Zeit bekannt. Solche Wirkstoffe sind beispielsweise in US-A- 4 127 405 oder EP-A- 44 807, 44 808 und 44 809 beschrieben.

In den Definitionen ist unter Alkyl geradkettiges oder verzweigtes Alkyl zu verstehen: z. B.: Methyl, Äthyl, n-Propyl, i-Propyl, die vier isomeren Butyl, n-Amyl, i-Amyl, 2-Amyl oder 3-Amyl.

Unter Alkoxy ist zu verstehen: Methoxy Äthoxy, n-Propyloxy, i-Propyloxy und die vier isomeren Butyloxyreste, insbesondere aber Methoxy, Äthoxy oder i-Propoxy.

Beispiele für Alkylthio sind Methylthio, Äthylthio, n-Propylthio, i-Propylthio und n-Butylthio, insbesondere aber Methylthio und Äthylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiel für Alkylsulfonyl sind Methylsulfonyl, Äthylsulfonyl, n-Propylsulfonyl und n-Butylsulfonyl, insbesondere aber Methylsulfonyl und Äthylsulfonyl.

Unter Halogen in den Definitionen sowie in Halogenalkyl, -alkoxy, -alkylsulfinyl, -alkylsulfonyl und -alkylthio sind Fluor, Chlor und Brom, vorzugsweise jedoch Fluor und Chlor zu verstehen.

Entsprechend sind unter Halogenalkyl bzw. Halogenalkylteilen von oben definierten Substituenten beispielsweise zu verstehen: Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Alkinylresten in den Definitionen der obigen Symbole entsprechen in der Regel Propargyl, 2-Butinyl, 3-Butinyl, sowie isomere Pentinyloder Hexinylreste, vorzugsweise ist der Alkinylrest jedoch durch Propargyl oder 2- oder 3-Butinyl verkörpert.

Unter die Definition der Gruppe X fallen sowohl solche Reste, in denen das Phosphoratom direkt an den Phenylkern gebunden ist, als auch solche, in denen zwischen Phenylkern und dem Phosphoratom eine Sauerstoff-, Schwefel-, Alkylen- oder Alkenylenbrücke besteht, wobei die Alkylenbrücken auch noch weitere Heteroatomglieder enthalten können. Bevorzugt sind solche Reste X, in denen das Phosphoratom entweder direkt oder über eine $C_2$-$C_3$-Alkylen- oder $C_2$-$C_3$-Alkenylenbrücke an den Phenylkern gebunden ist. Die funktionelle Phosphorgruppe kann dabei in verschiedenen Oxidationsstufen vorliegen, die folgenden zugrundeliegenden Phosphorsäuren entsprechen: phosphorige Säure, phosphorsäure, phosphonige Säure, Phosphonsäure, Phosphinige Säure und Phosphinsäure. Vorzugsweise stellen die Reste $R_4$ und $R_5$ jedoch $C_1$-$C_5$-Alkyl oder $C_1$-$C_5$-Alkoxy dar.

Die Erfindung umfasst ebenfalls die Salze, die die Verbindungen der Formel I mit Aminen, Alkali- und Erdalkalimetallbasen oder quaternären Ammoniumbasen bilden können.

Bewegliche Protonen, die zur Salzbildung leicht aus dem Molekül der Formel I ablösbar sind, befinden sich in der Phosphorsäuregruppe des Restes X, sofern freie Hydroxylgruppen vorhanden sind, oder am Brückenstickstoffatom zwischen Sulfonyl- und Carbonylgruppe.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Äthylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel sind die Verbindungen bevorzugt, in denen entweder

a) die Brücke $A_n$ eine direkte Bindung, $C_2$-$C_3$-Alkylen oder $C_2$-$C_3$-Alkenylen bedeutet oder
b) E Sauerstoff bedeutet, oder
c) $R^1$ Wasserstoff bedeutet oder
d) die Reste $R^2$ und $R^3$ für Methyl oder Methoxy stehen oder
e) G Sauerstoff bedeutet oder
f) Y Wasserstoff bedeutet.

Weiter bevorzugte Untergruppen von Verbindungen sind dadurch charakterisiert, dass die Brücke $A_n$ eine direkte Bindung, $C_2$-$C_3$-Alkylen oder $C_2$-$C_3$-Alkenylen, G Sauerstoff und $R^4$ und $R^5$ unabhängig voneinander $C_1$-$C_5$-Alkyl oder $C_1$-$C_5$-Alkoxy bedeuten oder dass E Sauerstoff, Y und $R^1$ Wasserstoff und $R^2$ und $R^3$ Methyl oder Methoxy bedeuten.

In einer ganz besonders bevorzugten Untergruppe zeichnen sich die umfassten Verbindungen dadurch aus, dass E und G Sauerstoff, Y und $R^1$ Wasserstoff, $R^2$ und $R^3$ Methyl oder Methoxy, die Brucke $A_n$ eine direkte Bindung, $C_2$-$C_3$-Alkylen oder $C_2$-$C_3$-Alkenylen und $R^4$ und $R^5$ $C_1$-$C_5$-Alkyl oder $C_1$-$C_5$-Alkoxy bedeuten.

Als bevorzugte Einzelverbindungen sind zu nennen:

N-[2-(Diäthoxyphosphonyl)-phenylsulfonyl]-N'-(4,6-dimethoxy-pyrimidin-2-yl)-harnstoff und

N-[2-(Di-n-butyloxyphosphonylvinyl)-phenylsulfonyl]-N'-(4-methoxy-6-methyl-pyrimidin-2-yl)-harnstoff.

Die Herstellung der Verbindungen der Formel I erfolgt im allgemeinen in einem inerten, organischen Lösungsmittel.

Nach einem ersten Verfahren werden die Verbindungen der Formel I erhalten, indem man ein Phenylsulfonamid der Formel II

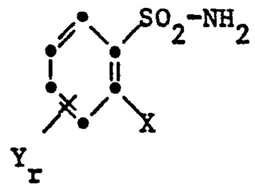

(II),

worin X, Y und r die unter Formel I gegebene Bedeutung haben, in Gegenwart einer Base mit einem N-Pyrimidinyl- oder -Triazinylcarbamat der Formel III

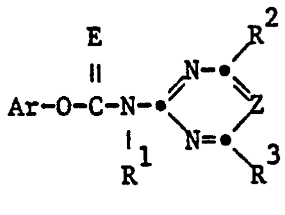

(III),

worin E, $R^1$, $R^2$, $R^3$ und Z die unter Formel I gegebene Bedeutung haben und Ar für Phenyl oder durch $C_1$-$C_5$-Alkyl, Halogen, $C_1$-$C_5$-Alkoxy oder Nitro substituiertes Phenyl steht, umsetzt.

Nach einem zweiten Verfahren gelangt man zu Verbindungen der Formel I, indem man ein Phenylsulfonylisocyanat oder -isothiocyanat der Formel IV

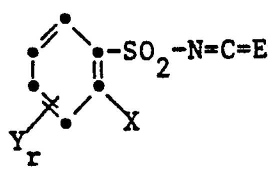

(IV),

worin

E, X, Y und r die unter Formel 1 gegebene Bedeutung haben, gegebenenfalls in Gegenwart einer Base, mit einem Amin der Formel V

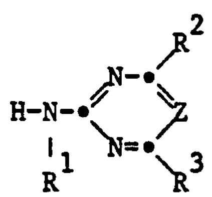

(V),

worin Z, $R^1$, $R^2$ und $R^3$ die unter Formel I gegebene Bedeutung haben, umsetzt.

Die erhaltenen Harnstoffe der Formel I können gewünschtenfalls mittels Aminen, Alkalimetall- oder Erdalkalimetallhydroxiden oder quaternären Ammoniumbasen in Salze übergeführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Äther wie Diäthyläther, Äthylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden. Als Basen sind insbesondere tertiäre Amine wie Trimethylamin, Triäthylamin, Chinuclidin, 1,4-Diazabicyclo-(2,2,2)-octan, 1,5-Diazabicyclo(4,3,0)non-5-en oder 1,5-Diazabicyclo(5,4,0)undec-7-en geeignet.

Die Endprodukte der Formel I können durch Einengen und/oder Verdämpfen des Lösungsmittels isoliert und durch Umkristallisieren oder Zerreiben des festen Rückstandes in Lösungsmitteln in denen sie sich nicht gut

lösen, wie Äther, aromatischen Kohlenwasserstoffen oder chlorierten Kohlenwasserstoffen gereinigt werden.

Die Ausgangsmaterialien der Formeln II und IV sind mit Ausnahme von 0-(2-Sulfamoylphenyl)-0,0-dimethylphosphat weches in. J. Med. Chem. 8, pp 377-385 (1965) beschrieben ist, neu. Diese Verbindungen wurden speziell für die Synthese der neuen Wirkstoffe der Formel I entwickelt. Die neuen Verbindungen der Formel II bilden einen Bestandteil der vorliegenden Erfindung.

Die Isocyanate oder Isothiocyanate der Formel IV sind nach an sich bekannten Methoden aus den Sulfonamiden der Formel II, beispielsweise durch Behandlung mit Phosgen oder Thiophosgen, erhältlich.

Die Ausgangsmaterialien der Formeln III und V sind bekannt oder können nach bekannten Methoden hergestellt werden.

Je nach der Beschaffenheit des ortho-Substituenten X lasaen sich die neuen phosphorhaltigen Sulfonamide der Formel II nach an sich bekannten Methoden herstellen. Zur Erläuterung solcher Herstellungsverfahren seien beispielhaft die folgenden Reaktionen erwähnt:

1) 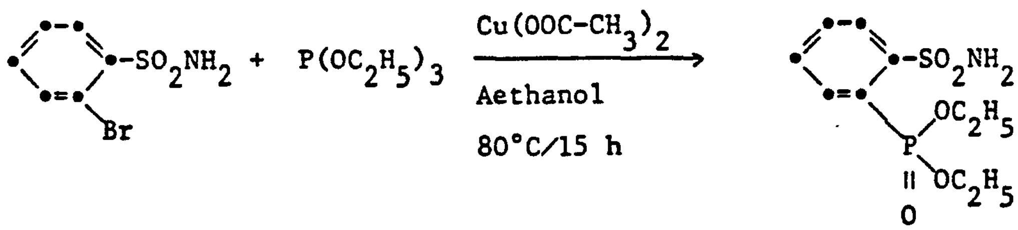


2) 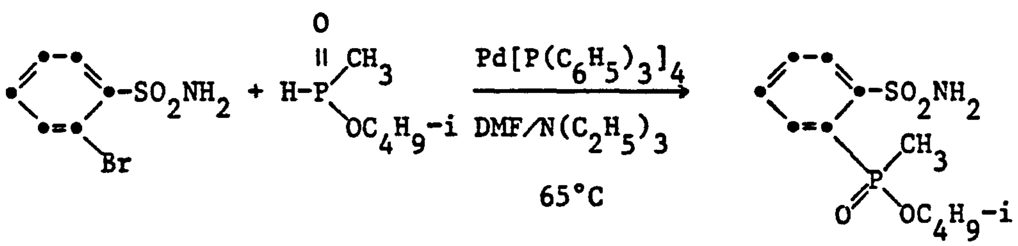


3) 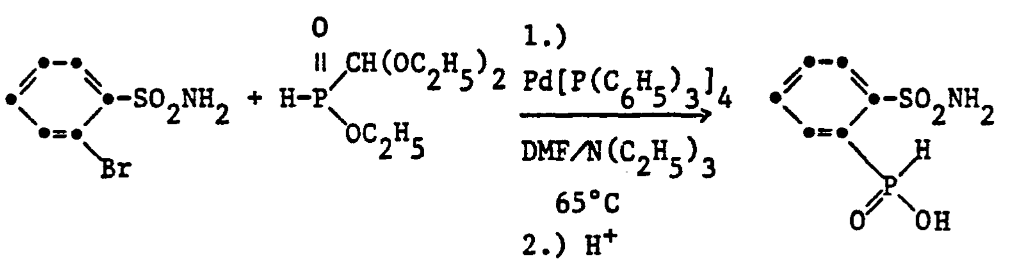


4) 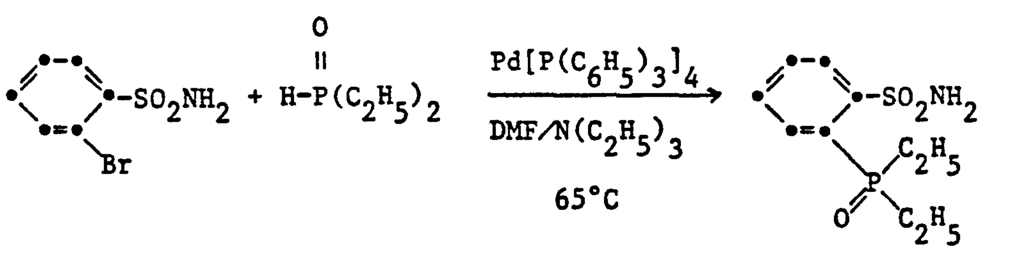


5) 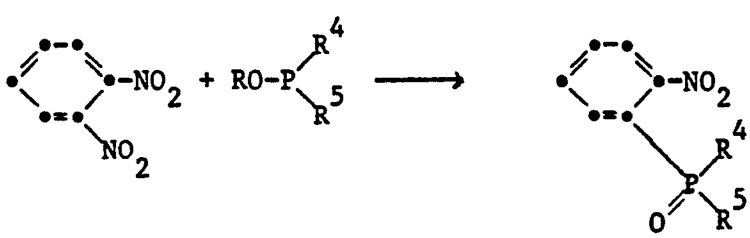

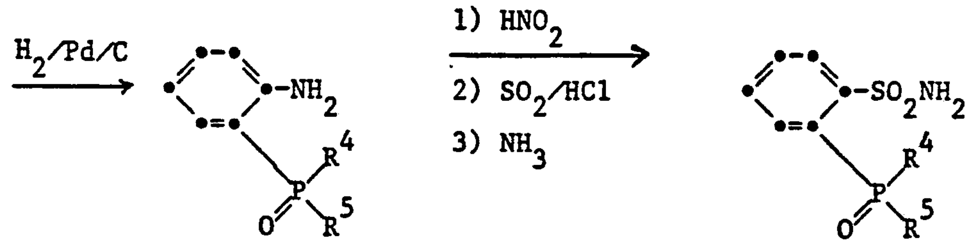


R = Alkyl
$R^4$, $R^5$ vorzugsweise $C_{1-5}$-Alkyl bzw.-Alkoxy

6) 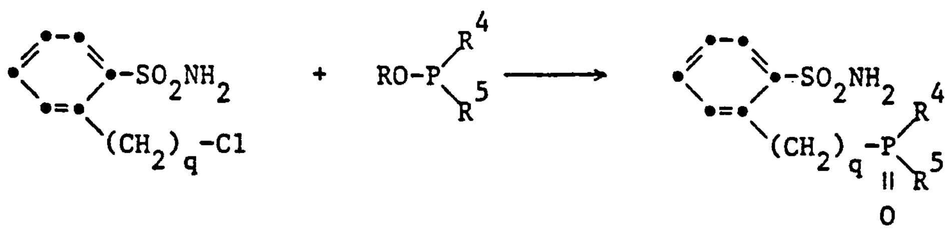

R = Alkyl, q = 1 bis 5
$R^4$, $R^5$ vorzugsweise $C_{1-5}$-Alkyl bzw.-Alkoxy

7) 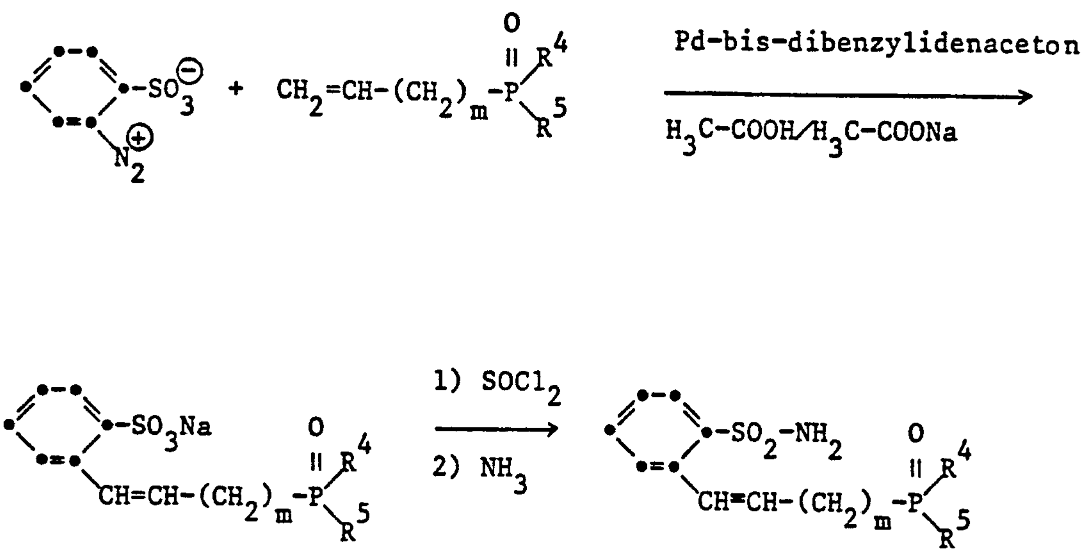


8) 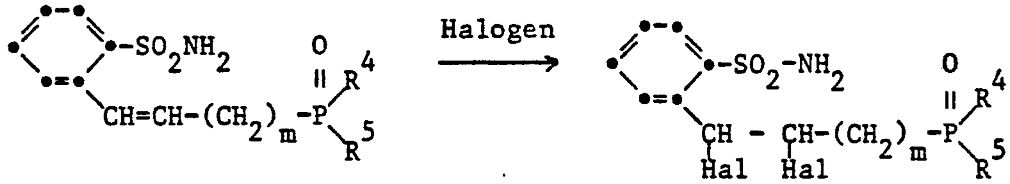

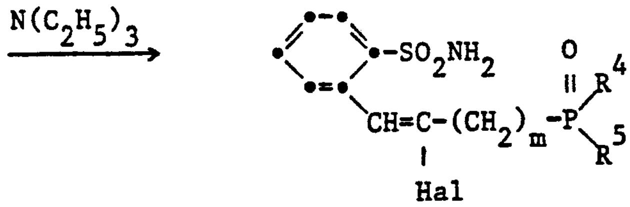


9) 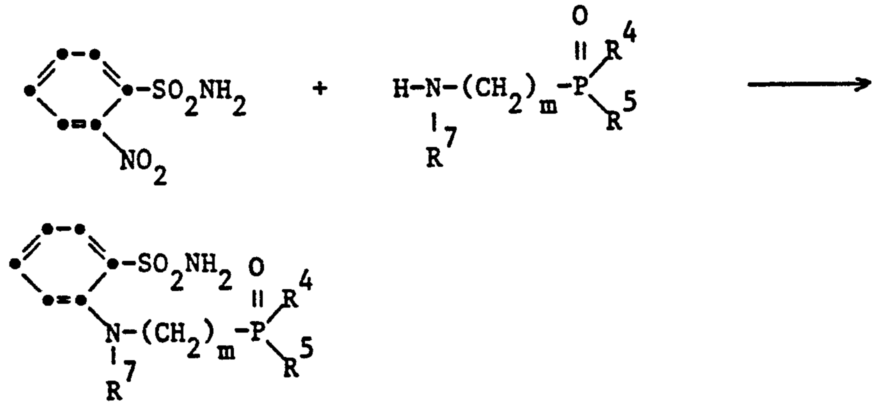

10) 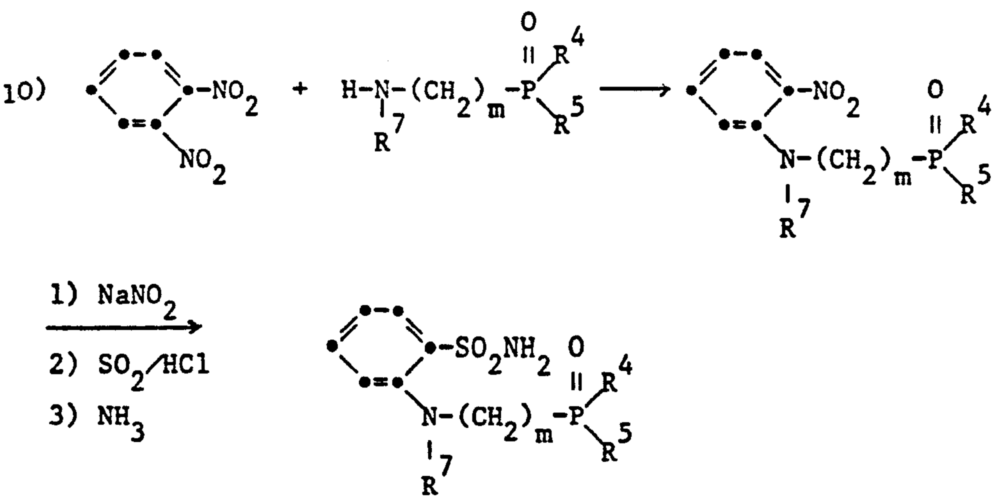


11) 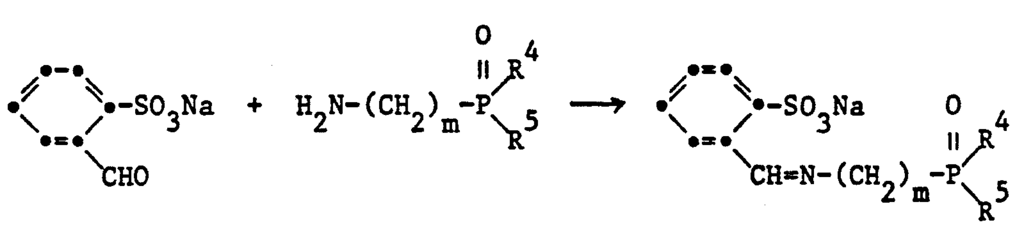

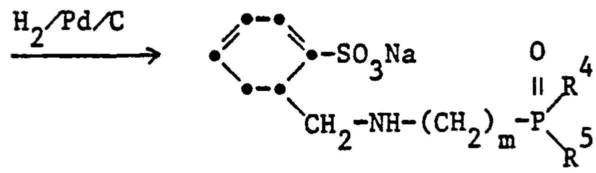


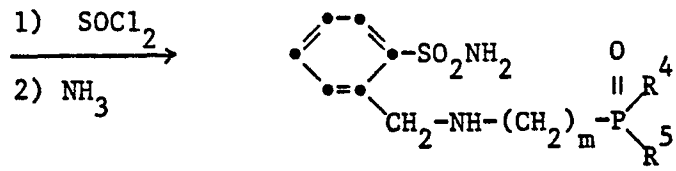


12) 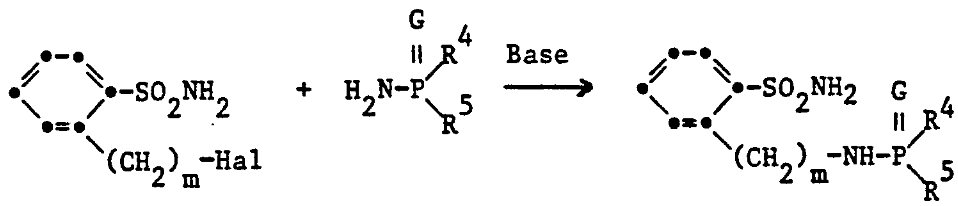


Hal = Halogen
$R^4$, $R^5$ vorzugsweise $C_{1-5}$-Alkyl bzw.-Alkoxy

13) 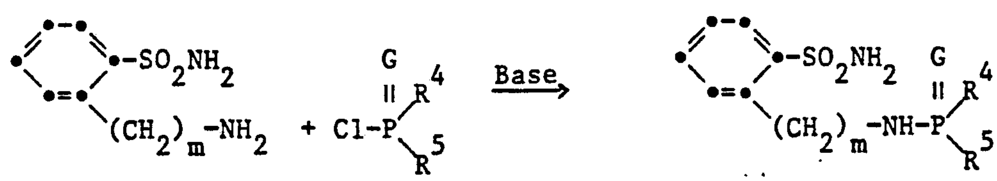


$R^4$, $R^5$ vorzugsweise $C_{1-5}$-Alkyl bzw.-Alkoxy

14) 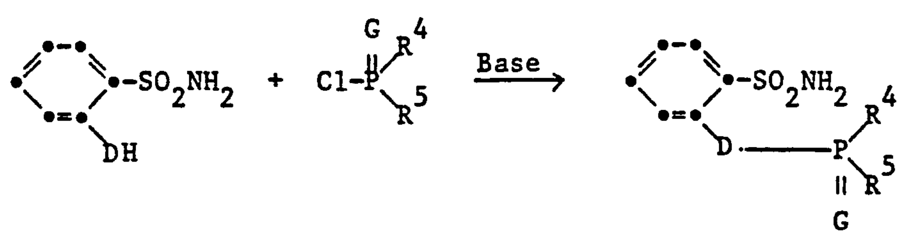


D = O, S
$R^4$, $R^5$ vorzugsweise $C_{1-5}$-Alkyl bzw.-Alkoxy

15) 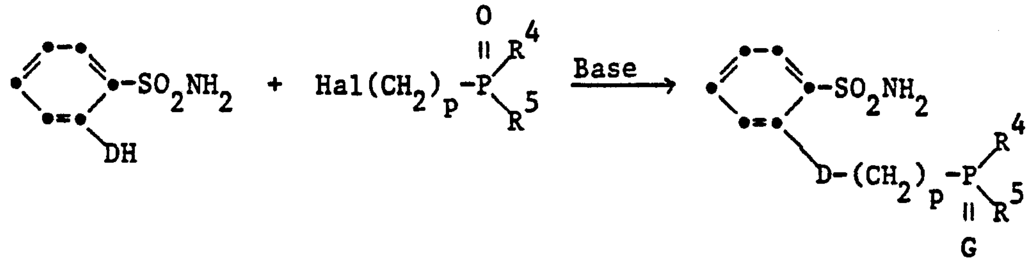


P = 0 bis 2

16) 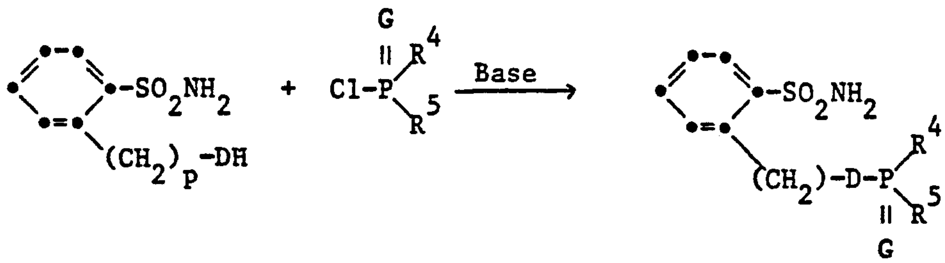


$R^4$, $R^5$ vorzugsweise $C_{1-5}$-Alkyl bzw.-Alkoxy

Die Wirkstoffe der Formel I sind stabile Verbindungen. Ihre Handhabung bedarf keine vorsorglichen Massnahmen.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflazen, insbesondere in Zuckerrohr, Getreide, Baumwolle, Soja, Mais und Reis befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen herbiziden und Wuchsregulatoren - sehr geringen Aufwandmengen.

Die Verbindungen der Formel I haben ausserdem starke pflanzenwuchsregulierende Eigenschaften, die sich in einer Ertragssteigerung von Kulturpflanzen oder Erntegut auswirken kann. Viele Verbindungen der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z. B. die in der Landwirtschaft in tropischen Gegenden häufig als "cover crop" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blütenund Fruchtbildung zugute kommen, während das vegetative Wachstum eingeschränkt wird.

Bei monokotylen Pflanzen z. B. Gräsern oder auch Kulturpflanzen wie Getreide, ist eine Hemmung des vegetativen Wachstums manchmal gewünscht und vorteilhaft. Eine derartige Wuchshemmung ist unter anderem bei Gräsern von wirtschaftlichem Interesse, denn dadurch kann z. B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen. Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten direkt versprühbaren oder verdünnbaren Lösungen verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Tragerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyloder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen Poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/ oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höhren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufig werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formildehydkondensationsproduktes.

Ferner kommen auch entsprechende Phosphate wie z. B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise ils Halogenide, Methylsulfate oder Äthylsulfate vor, z. B. das Stearyltrimethylammoniumchlorid oder das Benzyldi(2-chloräthyl)-äthylammoniumbromid.

Die in den Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J.Ash. "Encyclopedia of Surfactants", Vol. I-III, Chemical Publishing Co., New York, 1980-1981.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent) tEmulgierbare Konzentrate>k>k>k Aktiver Wirkstoff:>k 1 b>kis 20 %, bevorzugt 5 bis 10 %>k oberflächenaktive Mittel:>k 5 b>kis 30 %, vorzugsweise 10 bis 20 %>k flüssiges Trägermittel:>k 50 b>kis 94 %, vorzugsweise 70 bis 85 %.>k >k>k>k Stäube>k>k>k Aktiver Wirkstoff:>k 0,1 b>kis 10 %, vorzugsweise 0,1 bis 1 %>k festes Trägermittel:>k 99,9 b>kis 90 %, vorzugsweise 99,9 bis 99 %.>k >k>k>k Suspension-Konzentrate>k>k>k Aktiver Wirkstoff:>k 5 b>kis 75 %, vorzugsweise 10 bis 50 %>k Wasser:>k 94 b>kis 25 %, vorzugsweise 90 bis 30 %>k oberflächenaktives Mittel:>k 1 b>kis 40 %, vorzugsweise 2 bis 30 %.>k >k>k>k Benetzbare Pulver>k>k>k Aktiver Wirkstoff:>k 0,5 b>kis 90 %, vorzugsweise 1 bis 80 %>k oberflächeniktives Mittel:>k 0,5 b>kis 20 %, vorzugsweise 1 bis 15 %>k festes Trägermittel:>k 5 b>kis 95 %, vorzugsweise 15 bis 90 %.>k >k>k>k Granulate>k>k>k Aktiver Wirkstoff:>k 0,5 b>kis 30 %, vorzugsweise 3 bis 15 %>k festes Trägermittel:>k 99,5 b>kis 70 %, vorzugsweise 97 bis 85 %.>k>t

Während als Handelswerte eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden 0°C, die Drücke in Millibar mb angegeben.

**Herstellungsbeispiele:**

**Beispiel 1:**

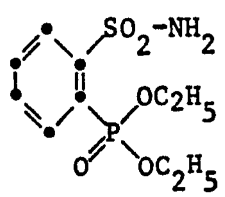

2-Diäthoxyphosphonyl-phenylsulfonamid.

59 g (0,25 Mol) 2-Bromphenylsulfonamid werden in 1 Liter Äthanol gelöst und mit 55 g (0,275 Mol) Kupfer-II-Acetat-Hydrat und 62 g (0,37 Mol) Triäthylphosphit versetzt und für 15 Stunden zum Rückfluss erhitzt. Dabei verfärbt sich die Lösung von Tiefblau nach Blaugrün. Nach dem Abkühlen wird die Lösung filtriert und eingedampft. Der Rückstand wird mit 200 ml Chloroform aufgenommen und 2 mal mit je 200 ml Wasser gewaschen. Nach dem Eindimpfen der organischen Phase wird der orangebraune Rückstand aus Äther kristallisiert. Man erhält so 16,3 g (22,3 % d.Th.) 2-Diäthoxyphosphonyl-phenylsulfonamid, Smp. 164-165°C.

**Beispiel 2:**

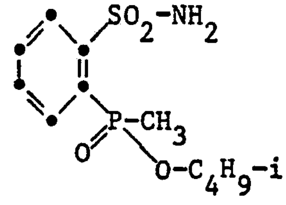

2-(i-Butyloxy-P-methyl-phosphinyl)-phenylsulfonamid.

Eine Lösung von 11,8 g (0,05 Mol) 2-Bromphenylsulfonsäureamid in 14 ml (0,1 Mol) Triäthylamin und 30 ml Dimethylformamid wird mit 6,8 g (0,05 Mol) Methylphosphinsäureisobutylester und 1,1 g (0,001 Mol) Tetrakistriphenylphosphinpalladium-Komplex versetzt. Die gelbe Suspension wird für 15 Stunden bei 100°C gerührt, abgekühlt und, filtriert. Nach dem Eindampfen des Filtrats wird der Rückstand aus Toluol kristallisiert. Man erhält so 4,12 g 2-(i-Butyloxy-P-methyl-phosphinyl)-phenylsulfonamid, Smp. 50-52°C.

**Beispiel 3**: 2-Diäthoxyphosphonyl-phenylsulfonamid.

a) 2-Diäthoxyphosphonyl-nitrobenzol:

Eine Mischung aus 63 g (0,375 Mol) 1,2-Dinitrobenzol und 131,2 ml (0,375 Mol) Triäthylphosphit wird in 350 ml Toluol für 7 Stunden zum Rückfluss erhitzt und dann eingedampft. Der ölige Rückstand wird im Vakuum fraktioniert. Die Fraktion mit einem Siedepunkt von 136-145°C/0,05 mbar wird in 100 ml Methylenchlorid aufgenommen und dann mit 200 ml Hexan versetzt. Die dabei ausfallenden Kristalle werden abgetrennt, mit Hexan gewaschen und getrocknet. Man erhält so 58,8 g (60,5 % d.Th.) 2-Diäthoxyphosphonyl-nitrobenzol als hellgelbe Kristalle, Smp. 56,5-57,5°C.

$^1$H-NMR ($CDCl_3$) :δ = 1,33 (t, $CH_3$, 6H); 4,23 (quintett, $OCH_2$, 4H); 7,5-8,3 (m, $C_6H_4$, 4H) ppm.

Analyse $C_{10}H_{14}NO_5P$ (259,2)

| | | | |
|---|---|---|---|
| berechnet | C 46,34 % | H 5,45 % | N 5,40 % |
| gefunden | C 46,2 % | H 5,4% | N 5,5 %. |

b) 2-Diäthoxyphosphonyl-anilin:

58,3 g 2-Diäthoxyphosphonyl-nitrobenzol werden in 590 ml Äthanol gelöst und über 3 g 5 %iger Palladium-Kohle bei 20-25°C mit Wasserstoff hydriert. Nach 20 Minuten sind von der Reaktion 104 % d.Th. Wasserstoff verbraucht und die Hydrierung ist beendet. Nach dem Abtrennen des Katalysators wird das Filtrat eingedampft. Durch Kristallisation des Rückstandes aus Hexan bei 0°C erhält man 48,2 g (42,7 % d.Th.) 2-Diäthoxyphosphonyl-anilin, Smp. 32-34°C.

$^1$H-NMR ($CDCl_3$): δ 1,30 (t, $CH_3$, 6H); 4,1 (quintett, $OCH_2$, 4H); 5,15 (s, $NH_2$, 2H); 6,5-7,7 (m, $C_6H_4$, 4H) ppm.

c) 2-Diäthoxyphosphonyl-phenylsulfonamid:

1,145 g 2-Diäthoxyphosphonyl-anilin, 1,05 ml 36 %iger Salzsäure und 0,78 ml Wasser werden unter Kühlung miteinander vermischt. Zur entstandenen Lösung lässt man innerhalb von 20 Minuten bei 0-5°C eine Lösung von 0,357 g Natriumnitrit in 0,537 ml Wasser zutropfen und rührt das Gemisch bei der gleichen Temperatur für 35 Minuten. Die entstandene Lösung wird synchron mit 0,95 ml einer wässrigen 40 %igen Natriumhydrogensulfitlösung innerhalb von 15 Minuten zu einem Gemisch aus 3,45 ml 36 %iger Salzsäure und 0,875 ml Wasser, 0,126 g (0,005 Mol) Kupfersulfathydrat und 0,95 ml wässriger 40 %iger Natriumhydrogensulfitlösung zugesetzt. Das Reaktionsgemisch wird für 1,5 Stunden bei 20-25°C gerührt und dreimil mit Methyenchlorid extrahiert. Die vereinigten orginischen Phasen werden zweimal mit wisser gewischen, über Natriumsulfat getrocknet und eingedampft. Man erhält so 1,5 g 2-(Diäthoxyphosphonyl-phenylsulfonylchlorid als Öl das ohne Reinigung mit 10,6 ml wässriger 30 %iger Ammoniaklösung bei 20-25°C umgesetzt wird. Der entstandene Niederschlag wird abgetrennt und mit Wasser gewaschen.

Ausbeute: 0,7 g 2-Diäthoxyphosphonyl-phenylsulfonamid, Smp. 161-162°C.

**Beispiel 4:**

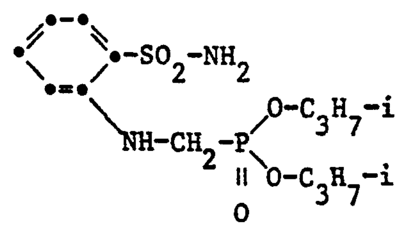

2-(Di-i-propyloxyphosphonymethylamino)-phenylsulfonamid.

10 g (0,58 Mol) 2-Nitrophenylsulfonamid werden in 20 ml Toloul suspendiert und zum Rückfluss erhitzt. In die eisse Lösung lässt man 17,0 g (0,087 Mol) Aminomethylphosponsäurediisopropylester zutropfen. Anschliessend wird die Reaktionsmischung für 10 Stunden am Rückfluss gekocht und nach dem Abkühlen eingedampft. Durch Chromatographie des Rückstandes an Kieselgel mit Methylacetat/Hexan-Gemisch (4:1) erhält man 1,6 g 2-(Di-i-propyloxyphosphonylmethylamino)-phenylsulfonamid als gelbes Öl.

$^1$H-NMR ($CDCl_3$): δ 1,30 (d, $CH_3$, 12H); 3,6 (2d, $J_PCH$ = 14Hz, $J_NHCH$ = 6Hz, 2H); 4,7 (m, OCH, 2H); 6,6-7,9 (m, $C_6H_4$, 4H); 8,0-8,3 (m, NH, $NH_2$, 3H) ppm.

**Beispiel 5:**

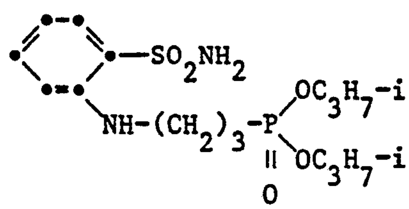

2-(Di-i-propyloxyphosphonylpropylamino)-phenylsulfonamid.

a) 2-(Di-i-propyloxyphosphonylpropylamino)-nitrobenzol:

Eine Mischung aus 20 g (0,11 Mol) 1,2-Dinitrobenzol und 31,87 g (0,14 Mol) Aminopropylphosphonsäurediisopropylester in 50 ml Toluol wird für 14 Stunden zum Rückfluss erhitzt und anschliessend eingedampft. Durch Chromatographie des Rückstands an Kieselgel mit Äthylacetat/Hexan-Gemisch (4:1) erhält man 32,04 g (84,6 % d.Th.) 2-(Di-i-propyloxyphosphonylpropylamino)-nitrobenzol als dunkelgelbes viskoses Öl.

$^1$H-NMR ($CDCl_3$): δ = 1,4 (d, $CH_3$, 12H); 2,0 (s, breit, $CH_2$, $CH_2P$, 4H); 3,5 (q, $NH_2$, 2H); 4,75 (m, OCH, 2H); 6,5-8,3 (s, breit, $C_6H_4$, NH, 5H) ppm.

b) 2-(Di-i-propyloxyphosphonylpropylamino)-anilin

Nach dem im Beispel 3a beschriebenen Verfahren erhält man aus dem Produkt des Beispiels 5a und Wasserstoff in Gegenwart von 5 % Pd/C als Produkt 2-(Di-i-propyloxyphosphonylpropylamino)-anilin als viskoses Öl.

**Beispiel 6:**

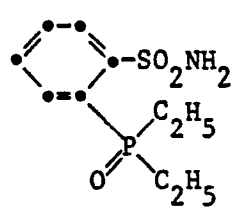

2-Diäthylphosphonyl-phenylsulfonimid.

Nach dem in Beispiel 2 beschriebenen Verfahren erhält man aus 2-Bromphenylsulfonamid und Diäthylphosphinoxid in Gegenwart von Triäthylamin, Dimethylformamid und Tetrakistriphenylphosphinpalladium-Komplex als Produkt 2-Diäthylphosphonyl-phenylsulfonamid, Smp. 139° C.

**Beispiel 7:**

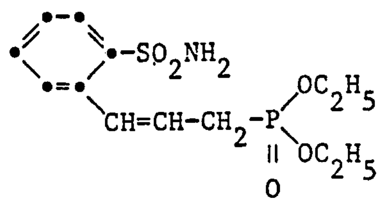

2-(3-Diäthoxyphosphonyl-1-propenyl)-phenylsulfonamid.

a) Ein Gemisch aus 34,64 g (0,2 Mol) Orthanilsäure, 30 ml Wasser und 62,3 ml 50 %iger Borfluorwasserstoffsäure (0,5 Mol) wird auf 0-5°C gekühlt und bei dieser Temperatur innerhalb von einer Stunde tropfenweise mit einer Lösung von 13,8 g (0,2 Mol) Natriumnitrit in 20 ml Wasser versetzt. Anschliessend wird die Mischung für 30 Minuten gerührt und mit 100 ml Äther aufgenommen. Der ausgefallene Niederschlag wird abgetrennt, mit 100 ml Essigsäure/Äther-Gemisch (1:1) und 100 ml Äther gewaschen. Nach dem Trocknen erhält man so 34 g Orthanilsäurediazoniumsalz (92 % d.Th.).

b) Ein Gemisch aus 8,65 g (0,05 Mol) Orthanilsäure, 50 ml Essigsäure und 6,25 ml (0,05 Mol) Borfluorwasserstoffsäure werden auf ca. 15°C gekühlt und bei dieser Temperatur innerhalb von 45 Minuten mit einer Lösung von 3,45 g (0,05 Mol) Natriumnitrit in 5 ml Wasser tropfenweise versetzt. Das entstandene Reaktionsgemisch wird bei 20-25°C für 30 Minuten gerührt. Die Suspension kann ohne weitere Isolierung des Diazoniumsalzes zur Weiterreaktion eingesetzt werden.

c) 7,73 g (0,042 Mol) des nach Beispiel 7a) hergestellten Diazoniumsalzes der Orthanilsäure werden in 50 ml Essigsäure ausgeschlämmt und mit 3,44 g (0,042 Mol) Natriumacetat und 0,1265 g (0,00021 Mol) Palladiumdibenzylidenaceton versetzt. Zu diesem Gemisch lässt man bei 20-25°C 7,85 g (0,044 Mol) Propenyl-3-phosphonsäurediäthylester zutropfen. Die Reaktion setzt unter Erwärmung und Stickstoffentwicklung ein. Am Ende der Gasentwicklung wird die Reaktionsmischung noch für 1,5 Stunden gerührt. Anschliessend wird das Lösungsmittel vollständig entfernt. Der erhaltene Rückstand wird in 50 ml Dimethylformamid aufgenommen und bei einer Temperatur von 5-10°C tropfenweise mit 7,64 ml (0,105 Mol) Thionylchlorid versetzt. Nach einer Reaktionszeit von 2 Stunden wird das Gemisch auf Eis gegossen und mit Äthylacetat extrahiert. Die vereinigten organischen Phasen werden mit Wisser gewischen und anschliessend tropfenweise einem Gemisch von 25 ml konzentriertem Ammoniak und Eis zugesetzt. Nach dem Abschluss der Reaktion wird die Mischung mit Wasser verdünnt und die organische Phase abgetrennt. Durch Chromatographieren an Kieselgel mit Toluol/Äthylacetat-Gemisch (1:1) erhält man 2,28 g (16 % d.Th.) 2-(3-Diäthoxyphosphonyl-1-propenyl)-phenylsulfonamid, Smp. 103-104°C.

Analyse $C_{13}H_{20}NO_5PS$ (333,34)

| | | | | | |
|---|---|---|---|---|---|
| berechnet | C 46,85 % | H 6,05 % | N 4,20 % | P 9,30 % | S 9,62 % |
| gefunden | C 46,93 % | H 6,04 % | N 4,13 % | P 9,25 % | S 9,38 %. |

**Beispiel 8:**

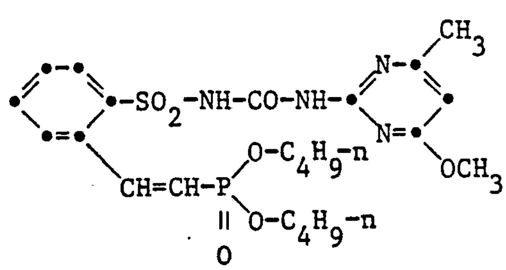

N-[2-(Di-n-butyloxyphosphonylvinyl)-phenylsulfonyl]-N'-(4-methoxy-6-methyl-pyrimidin-2-yl)-harnstoff. (Verbindung 4.1 ).

a) 2-(Di-n-butyloxyphosphonylvinyl)-phenylsulfonamid.

11,04 g (0,06 Mol) nach der Vorschrift von Beispiel 7a) erhaltenes Diazoniumsalz werden in 70 ml Essigsäure suspendiert und in Gegenwart von 4,29 g (0,06 Mol) Natriumacetat und 0,182 g (0,003 Mol) Palladiumdibenzylidenaceton mit 13,2 g Vinylphosphonsäure-di-n-butylester versetzt. Nach Beendigung der

Stickstoffentwicklung wird das Gemisch für 1/2 Stunden weitergerührt. Anschliessend wird das Lösungsmittel abgedampft und der Rückstand in 40 ml Dimethylformamid aufgenommen und unter Zusatz von 10,91 ml (0,15 Mol) Thionylchlorid bei 5°C für 1,5 Stunden gerührt. Anschliessend wird das Reaktionsgemisch auf Eis gegossen und mit Äthylacetat extrahiert. Die vereinigten Äthylacetatphasen werden tropfenweise zu 20 ml konzentrierter Ammoniaklösung und 20 g Eis getropft. Nach Abschluss der Reaktion wird die organische Phase abgetrennt, mit Wasser gewaschen, getrocknet und eingedampft. Durch Chromatographie des öligen Rückstands an Kieselgel mit Toluol/Äthylacetat-Gemisch (4:1) erhält man 13,72 g (61 % d.Th.) 2-(Di-n-butyloxyphosphonylvinyl)-phenylsulfonamid als viskoses Öl.

Analyse $C_{16}H_{26}NO_5PS$ (375,42)

| | | | | | |
|---|---|---|---|---|---|
| berechnet | C 51,19 % | H 6,98 % | N 3,73 % | P 8,25 % | S 8,54 % |
| gefunden | C 51,28 % | H 7,09 % | N 3,60 % | P 8,02 % S | 8,41 %. |

b) Einer Lösung von 5,63 g (0,015 Mol) 2-(Di-n-butyloxyphosphonylvinyl)-phenylsulfonamid in 80 ml Dioxan setzt man bei 20-25°C 2,4 ml (0 0165 Mol) 1,5-Diazabicyclo(5,4,0)undec-5-en sowie 3,9 g (0,015 Mol) N-(4-methoxy-6-methyl-pyrimidin-2-yl)-phenylcarbamat zu. Nachdem das Gemisch für 2 Stunden gerührt worden ist, wird es auf 200 ml Wasser gegossen. Die entstehende klare Lösung wird mit 36 %iger Salzsäure bis auf einen pH Wert 2 angesäuert und mit Methylacetat extrahiert. Die vereinigten organischen Phasen werden getrocknet und eingedampft. Durch Kristallisation des orangen öligen Rückstands aus Petroläther erhält man 6,9 g (85,2 % d.Th.) N-[2-(Di-n-butyloxyphosphonylvinyl)-phenyl-sulfonyl]-N'-(4-methoxy-6-methyl-pyrimidin-2-yl)-harnstoff, Smp. 86-87°C.

**Beispiel 9:**

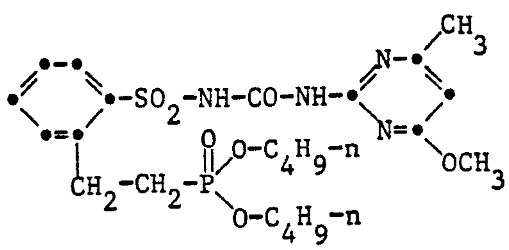

N-[2-(Di-n-butyloxyphosphonyläthyl)-phenylsulfonyl]-N'-(4-methoxy-6-methyl-pyrimidin-2-yl)-harnstoff (Verbindung 4.21).

4,0 g (0 074 Mol) N-[2-(Di-n-butyloxyphosphonylvinyl)-phenylsulfonyl]-N'-(4-methoxy-6-methyl-pyrimidin-2-yl)-harnstoff werden in 40 ml Tetrahydrofuran gelöst und unter Zugabe von 0,5 g Platinhydroxyd bei 20-25°C mit Wasserstoff hydriert. Nach einer Aufnahme von 114 % der theoretisch benötigten Wasserstoffmenge kommt die Reaktion zum Stillstand. Der Katalysator wird abfiltriert und die Lösung eingedampft. Der farblose Rückstand wird aus Äther kristallisiert. Man erhält so 4,0 g (100 % d.Th.) N-[2-(Di-n-butyloxyphosphonyläthyl)-phenylsulfonyl]-N'-(4-methoxy-6-methyl-pyrimidin-2-yl)-harnstoff, Smp. 97-98°C.

**Beispiel 10:**

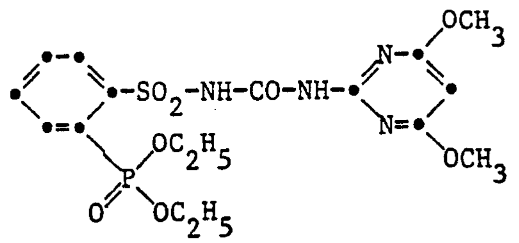

N-(2-Diäthoxyphosphonyl-phenylsulfonyl)-N'-(4,6-dimethoxy-pyrimidin-2-yl)-harnstoff. (Verbindung 4.31).

1,7 g 2-Diäthoxyphosphonyl-phenylsulfonamid und 0,9 g 1,5-Diazabicyclo(5,4,0)undec-5-en werden in 19 ml Dioxan gelöst und mit 1,6 g N-(4,6-dimethoxy-pyrimidin-2-yl)-phenylcarbamat versetzt. Nachdem die Lösung für 5 Stunden bei 20-25°C gerührt worden ist werden 100 ml Äthylacetat zugesetzt. Die Lösung wird durch

Zugabe von 2N Salzsäure bis auf einen pH Wert von 2 angesäuert. Dabei fällt das Produkt teilweise aus. Die wässrige Phase wird abgetrennt, die organische Phase zweimal mit Wasser gewaschen. Nach dem Eindampfen der organischen Phase kristallisiert man den Rückstand aus einem Aceton/Äther -Gemisch. Man erhält 2,5 g (90,8 % d.Th.) N-(2-Diäthoxyphosphonyl-phenylsulfonyl)-N'-(4,6-dimethoxy-pyrimidin-2-yl)-harnstoff, Smp. 172° C (Zers.).

Analyse $C_{17}H_{23}N_4O_8PS$ (474,43)

| | | | | | |
|---|---|---|---|---|---|
| berechnet | C 43,04 % | H 4,89 % | N 11,81 % | P 6,53 % | S 6,76 % |
| gefunden | C 43,5 % | H 4,6 % | N 12,1 % | P 6,6 % | S 6,6 %. |

In analoger Weise erhält man die in den anschliessenden Tabellen aufgelisteten Zwischen- und Endprodukte.

**Tabelle 1:**

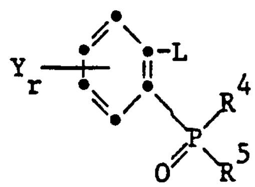

| Nr. | $R_4$ | $R_5$ | L | Y | r | phys. Daten |
|---|---|---|---|---|---|---|
| 1.1 | $OC_2H_5$ | $OC_2H_5$ | $NO_2$ | H | 1 | Smp. 56,5-57,5° C |
| 1.2 | $OC_2H_5$ | $OC_2H_5$ | $NH_2$ | H | 1 | Smp. 32-34° C |
| 1.3 | $0C_2H_5$ | $CH_3$ | $NO_2$ | H | 1 | Spd. 129° C/0,08 mb |
| 1.4 | $OC_2H_5$ | $OC_2H_5$ | $NO_2$ | 5-Cl | 1 | Smp. 61-62° C |
| 1.5 | $OC_2H_5$ | $CH_3$ | $NO_2$ | 5-Cl | 1 | Sdp. 151-152°/0,26 mb |
| 1.6 | OH | OH | $NO_2$ | H | 1 | Smp. 197-199° C |
| 1.7 | OH | $CH_3$ | $NO_2$ | H | 1 | Smp. 147-149° C |
| 1.8 | OH | OH | $NO_2$ | 5-Cl | 1 | Smp. 208-210° C |
| 1.9 | OH | $CH_3$ | $NO_2$ | 5-Cl | 1 | Smp- 150-151° C |
| 1.10 | $OC_2H_5$ | $CH_3$ | $NH_2$ | 4-$CF_3$, 6-$NO_2$ | 2 | Smp. 117-119° C |
| 1.11 | $OC_2H_5$ | $CH_3$ | $NO_2$ | 4-$CF_3$, 6-$NO_2$ | 2 | Smp. 130-132° C |
| 1.12 | $C_6H_5$ | OH | $NO_2$ | 4-$CF_3$, 6-$NO_2$ | 2 | Smp. 133-138 ° C |
| 1.13 | $C_6H_5$ | $OC_4H_9$-n | $NO_2$ | 4-$CF_3$, 6-$NO_2$ | 2 | Smp. 89-90° C |
| 1.14 | $C_6H_5$ | $C_6H_5$ | $NO_2$ | 4-$CF_3$, 6-$NO_2$ | 2 | Smp. I88-190° C |
| 1.15 | $OC_2H_5$ | $OC_2H_5$ | $NH_2$ | 5-Cl | 1 | Smp. 72-75° |
| 1.17 | $OC_2H_5$ | $OC_2H_5$ | $NO_2$ | 4-$COOC_2H_5$ | 1 | braunes Öl |
| 1.19 | OH | OH | $NO_2$ | 4-$COOC_2H_5$ | 1 | Smp. 184-186° C |

**Tabelle 2**:

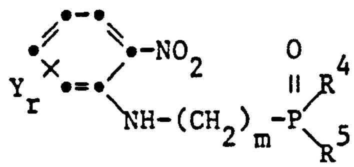

| Nr. | $R^4$ | $R^5$ | Y | r | m | phys. Daten |
|---|---|---|---|---|---|---|
| 2.1 | $OC_3H_7$-i | $OC_3H_7$-i | H | 1 | 3 | gelbes Öl |
| 2.2 | $OC_2H_5$ | $OC_2H_5$ | H | 1 | 1 | Smp. 81-83°C |
| 2.3 | OH | OH | H | 1 | 3 | Smp. 142-145°C |
| 2.4 | OH | OH | H | 1 | 1 | Smp. 147-148°C |
| 2.5 | $OC_2H_5$ | $OC_2H_5$ | H | 1 | 2 | oranges Öl |
| 2.6 | $OC_2H_5$ | $OC_2H_5$ | 5-Cl | 1 | 1 | oranges Öl |
| 2.7 | $OC_2H_5$ | $OC_2H_5$ | 5-Cl | I | 2 | hochviskoses Öl |
| 2.8 | OH | OH | H | 1 | 2 | Smp. 124-127°C |
| 2.9 | $OC_3H_7$-i | $OC_3H_7$-i | 5-Cl | 1 | 3 | viskoses Öl |
| 2.10 | OH | OH | 5-Cl | 1 | 3 | Smp. 218°C |
| 2.11 | $OC_3H_7$-i | $OC_3H_7$-i | 4-$COOC_2H_5$ | 1 | 1 | Smp. 73-74°C |
| 2.13 | OH | OH | 4-$COOC_2H_5$ | 1 | 1 | gelbes Öl |

**Tabelle 3**:

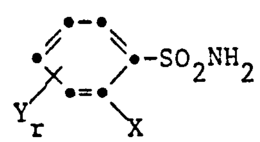

| Nr. | X | Y | r | phys. Daten |
|---|---|---|---|---|
| 3.1 | $-PO(OC_2H_5)_2$ | H | 1 | Smp. 164-165°C |
| 3.2 | $-PO(CH_3)-OC_4H_9$-i | H | 1 | Smp. 50-52°C |
| 3.3 | $-PO(C_2H_5)_2$ | H | 1 | Smp. 139°C |
| 3.4 | $-CH=CH-CH_2-PO(OC_2H_5)_2$ | H | 1 | Smp. 103-104°C |
| 3.5 | $-CH=CH-PO(OC_4H_9\text{-n})_2$ | H | 1 | viskoses Öl |
| 3.6 | $-(CH_2)_3-PO(OC_2H_5)_2$ | H | 1 | |
| 3.7 | $-(CH_2)_2-PO(OC_2H_5)_2$ | H | 1 | |
| 3.8 | $-CH_2-PO(OC_2H_5)_2$ | H | 1 | Smp. 110-117°C |
| 3.9 | $-NH-CH_2-PO(OC_3H_7\text{-i})_2$ | H | 1 | |
| 3.10 | $-NH-(CH_2)_2-PO(OC_3H_7\text{-i})_2$ | H | 1 | |
| 3.11 | $-NH-(CH_2)_3-PO(OC_3H_7\text{-i})_2$ | H | 1 | |
| 3.12 | $-NH-(CH_2)_2-PO(OC_2H_5)_2$ | H | 1 | |
| 3.13 | $-PO(OC_2H_5)_2$ | 5-Cl | 1 | |
| 3.14 | $-PO(CH_3)-OC_2H_5$ | 5-Cl | 1 | |
| 3.15 | $-PO(CH_3)-OC_2H_5$ | H | 1 | |
| 3.16 | $-PO(OH)_2$ | H | 1 | |
| 3.17 | $-PO(OH)_2$ | 5-Cl | 1 | |
| 3.18 | $-PO(OC_2H_5)_2$ | 4-$CF_3$, 6-$NO_2$ | 2 | |
| 3.19 | $-PO(CH_3)-OC_2H_5$ | 4-$CF_3$, 6-$NH_2$ | 2 | |
| 3.20 | $-PO(CH_3)-OC_2H_5$ | 4-$CF_3$, 6-$NO_2$ | 2 | |
| 3.21 | $-PO(OC_2H_5)_2$ | 4-$COOC_2H_5$ | 1 | |
| 3.22 | $-NH-(CH_2)_3-PO(OC_2H_5)_2$ | 5-Cl | 1 | |
| 3.23 | $-NH-(CH_2)_3-PO(OC_3H_7\text{-i})_2$ | 5-Cl | 1 | |
| 3.24 | $-NH-CH_2-PO(OC_2H_5)_2$ | H | 1 | |

**Tabelle 4:**

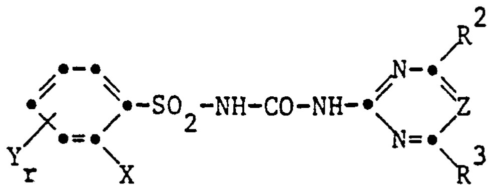

| Nr. | $R^2$ | $R^3$ | X | Y | r | Z | phys. Daten |
|---|---|---|---|---|---|---|---|
| 4.1 | $CH_3$ | $OCH_3$ | $-CH=CH-PO(OC_4H_9\text{-}n)_2$ | H | 1 | CH | Smp. 86-87° |
| 4.2 | $CH_3$ | $CH_3$ | $-CH=CH-PO(OC_4H_9\text{-}n)_2$ | H | 1 | CH | |
| 4.3 | $CH_3$ | $OCH_3$ | $-CH=CH-PO(OC_4H_9\text{-}n)_2$ | H | 1 | N | |
| 4.4 | $OCH_3$ | $OCH_3$ | $-CH=CH-PO(OC_2H_9\text{-}n)_2$ | H | 1 | N | |
| 4.5 | $OCH_3$ | $OCH_3$ | $-CH=CH-PO(OC_2H_9\text{-}n)_2$ | H | 1 | CH | |
| 4.6 | $CH_3$ | $OCH_3$ | $-CH=CH-CH_2-PO(OC_2H_5)_2$ | H | 1 | N | Smp. 153-154° C |
| 4.7 | $OCH_3$ | $OCH_3$ | $-CH=CH-CH_2-PO(OC_2H_5)_2$ | H | 1 | N | |
| 4.8 | $CH_3$ | $OCH_3$ | $-CH=CH-CH_2-PO(OC_2H_5)_2$ | H | 1 | CH | |
| 4.9 | $CH_3$ | $OCH_3$ | $-CH=CH-CH_2-PO(OC_2H_5)_2$ | H | 1 | CH | |
| 4.10 | $OCH_3$ | $OCH_3$ | $-CH=CH-CH_2-PO(OC_2H_5)_2$ | H | 1 | CH | |
| 4.11 | $CH_3$ | $OCH_3$ | $-PO(CH_3)-OC_4H_9\text{-}i$ | H | 1 | CH | |
| 4.12 | $CH_3$ | $CH_3$ | $-PO(CH_3)-OC_4H_9\text{-}i$ | H | 1 | CH | |
| 4.13 | $OCH_3$ | $OCH_3$ | $-PO(CH_3)-OC_4H_9\text{-}i$ | H | 1 | CH | |
| 4.14 | $CH_3$ | $OCH_3$ | $-PO(CH_3)-OC_4H_9\text{-}i$ | H | 1 | N | |
| 4.15 | $OCH_3$ | $OCH_3$ | $-PO(CH_3)-OC_4H_9\text{-}i$ | H | 1 | N | |
| 4.16 | $CH_3$ | $OCH_3$ | $-PO(C_2H_5)_2$ | H | 1 | CH | |
| 4.17 | $OCH_3$ | $OCH_3$ | $-PO(C_2H_5)_2$ | H | 1 | CH | |
| 4.18 | $CH_3$ | $CH_3$ | $-PO(C_2H_5)_2$ | H | 1 | CH | |
| 4.19 | $CH_3$ | $OCH_3$ | $-PO(C_2H_5)_2$ | H | 1 | N | |
| 4.20 | $OCH_3$ | $OCH_3$ | $-PO(C_2H_5)_2$ | H | 1 | N | |
| 4.21 | $CH_3$ | $OCH_3$ | $-(CH_2)_2-PO(OC_4H_9\text{-}n)_2$ | H | 1 | CH | Smp. 97-98° C |
| 4.22 | $CH_3$ | $CH_3$ | $-(CH_2)_2-PO(OC_4H_9\text{-}n)_2$ | H | 1 | CH | |
| 4.23 | $OCH_3$ | $OCH_3$ | $-(CH_2)_2-PO(OC_4H_9\text{-}n)_2$ | H | 1 | CH | |
| 4.24 | $OCH_3$ | $OCH_3$ | $-(CH_2)_2-PO(OC_4H_9\text{-}n)_2$ | H | 1 | N | |
| 4.25 | $OCH_3$ | $OCH_3$ | $-(CH_2)_2-PO(OC_4H_9\text{-}n)_2$ | H | 1 | N | |
| 4.26 | $CH_3$ | $OCH_3$ | $-(CH_2)_3-PO(OC_2H_5)_2$ | H | 1 | CH | |
| 4.27 | $OCH_3$ | $OCH_3$ | $-(CH_2)_3-PO(OC_2H_5)_2$ | H | 1 | CH | |
| 4.28 | $CH_3$ | $CH_3$ | $-(CH_2)_3-PO(OC_2H_5)_2$ | H | 1 | CH | |
| 4.29 | $CH_3$ | $OCH_3$ | $-(CH_2)_3-PO(OC_2H_5)_2$ | H | 1 | N | |
| 4.30 | $OCH_3$ | $OCH_3$ | $-(CH_2)_3-PO(OC_2H_5)_2$ | H | 1 | N | |
| 4.31 | $OCH_3$ | $OCH_3$ | $-PO(OC_2H_5)_2$ | H | 1 | CH | Smp. 172° (Zers.) |
| 4.32 | $OCH_3$ | $OCH_3$ | $-PO(OC_2H_5)_2$ | H | 1 | CH | Smp. 172° C (Zers.) |
| 4.33 | $CH_3$ | $OCH_3$ | $-PO(OC_2H_5)_2$ | H | 1 | CH | |
| 4.34 | $CH_3$ | $CH_3$ | $-PO(OC_2H_5)_2$ | H | 1 | CH | |
| 4.35 | $CH_3$ | $OCH_3$ | $-PO(OC_2H_5)_2$ | H | 1 | N | |
| 4.36 | $OCH_3$ | $OCH_3$ | $-PO(OC_2H_5)_2$ | H | 1 | N | |
| 4.37 | $OCH_3$ | $OCH_3$ | $-PO(CH_3)_2$ | H | 1 | CH | |
| 4.38 | $CH_3$ | $OCH_3$ | $-PO(CH_3)_2$ | H | 1 | CH | |
| 4.39 | $OCH_3$ | $OCH_3$ | $-PO(CH_3)_2$ | H | 1 | CH | |
| 4.40 | $CH_3$ | $OCH_3$ | $-PO(CH_3)_2$ | H | 1 | N | |
| 4.41 | $OCH_3$ | $OCH_3$ | $-PO(CH_3)_2$ | H | 1 | N | |
| 4.42 | $CH_3$ | $OCH_3$ | $-CH_2-PO(OC_2H_5)_2$ | H | 1 | N | Smp. 120-125° C (Zers.) |
| 4.43 | $CH_3$ | $OCHF_2$ | $-NHCH_2-PO(OC_3H_7)\text{-}i_2$ | H | 1 | CH | Smp. 109-111 °C (Zers.) |
| 4.44 | $CH_3$ | $OCH_3$ | $-NHCH_2-PO(OC_3H_7\text{-}i)_2$ | H | 1 | CH | Smp. 130-135° C (Zers.) |
| 4.45 | $CH_3$ | $OCH_3$ | $-CH_2-S-PS(OC_2H_5)_2$ | H | 1 | N | Smp. 125-127° C |
| 4.46 | $CH_3$ | $OCH_3$ | $-CH_2-S-PS(OC_2H_5)_2$ | H | 1 | CH | Smp. 145-147° C |

**Formulierungsbeispiele**

**Beispiel 11:**
Formulierungsbeispiele für Wirkstoffe der Formel I (% = Gewichtsprozent)

| | | a) | b) | c) |
|---|---|---|---|---|
| a) | Spritzpulver | | | |
| | Wirkstoff | 20 % | 60 % | 0,5 % |
| | Na-Ligninsulfonat | 5 % | 5 % | 5 % |
| | Na-Laurylsulfat | 3 % | - | - |
| | Na-Diisobutylnaphthalinsulfonat | - | 6 % | 6 % |
| | Octylphenolpolyäthylenglykoläther (7-8 Mol ÄO) | - | 2 % | 2 % |
| | Hochdisperse Kieselsäure | 5 % | 27 % | 27 % |
| | Kaolin | 67 % | - | - |
| | Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) | Emulsion-Konzentrat | a) | b) |
|---|---|---|---|
| | Wirkstoff | 10 % | 1 % |
| | Octylphenolpolyäthylenglykoläther (4-5 Mol ÄO) | 3 % | 3 % |
| | Ca-Dodecylbenzolsulfonat | 3 % | 3 % |
| | Ricinusölpolyglykoläther (36 Mol ÄO) | 4 % | 4 % |
| | Cyclohexanon | 30 % | 10 % |
| | Xylolgemisch | 50 % | 79 % |

Aus diesem Konzentrat können durch Verdünnen mit Wasser Emulsionen jeder gewünschten Konzentration hergestellt werden.

| c) | Stäubemittel | a) | b) |
|---|---|---|---|
| | Wirkstoff | 0,1 % | 1 % |
| | Talkum | 99,9 % | - |
| | Kaolin | - | 99 % |

Man erhält anwendungsfertige Stäubemittel, indem der Wirkstoff mit dem Träger vermischt und auf einer geeigneten Mühle vermahlen wird.

| d) | Extruder Granulat | a) | b) |
|---|---|---|---|
| | Wirkstoff | 10 % | 1 % |
| | Na-Ligninsulfonat | 2 % | 2 % |
| | Carboxymethylcellulose | 1 % | 1 % |
| | Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) | Umhüllungs-Granulat | a) |
|---|---|---|
| | Wirkstoff | 3 % |
| | Polyäthylenglykol (MG 200) | 3 % |
| | Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) | Suspensions-Konzentrat | a) | b) |
|---|---|---|---|
| | Wirkstoff | 40 % | 5 % |
| | Äthylenglykol | 10 % | 10 % |
| | Nonylphenolpolyäthylenglykoläther (15 Mol ÄO) | 6 % | 1 % |
| | Na-Ligninsulfonat | 10 % | 5 % |
| | Carboxymethylcellulose | 1 % | 1 % |
| | 37 %ige wässrige Formaldehyd-Lösung | 0,2 % | 0,2 % |
| | Silikonöl in Form einer 75 %igen wässrigen Emulsion | 0,8 % | 0,8 % |
| | Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | a) |
|---|---|
| Wirkstoff | 5 % |
| Isopropylamin | 1 % |
| Octylphenolpolyäthylenglykoläther (78 Mol ÄO) | 3 % |
| Wasser | 91 % |

**Beispiel 12:**

Herbizidwirkung vor dem Auflaufen der Pflanzen Kunststofftöpfe werden mit expandiertem Vermiculit (Dichte: 0,135 g/$cm^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5 % eines handelsüblichen Flüssigdüngers (®Greenzit) augesetzt. 12 Tage nach der Aussaat wird der Versuch zusgewertet und die Wirkung auf die Versuchspflanzen nach dem folgenden Masstab bewertet:

1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:
Konzentration der Wirkstoffemulsion: 70,8 ppm

| Testpflanze<br>Wirkstoff Nr. | Nasturtium | Stellaria | Agrostis | Digitaria |
|---|---|---|---|---|
| 4.6 | 3 | 6 | 3 | 8 |
| 4.31 | 2 | 1 | 1 | 3 |
| 4.45 | 2 | 2 | 2 | 2 |
| 4.46 | 2 | 2 | 1 | 2 |

**Beispiel 13:**

Herbizidwirkung nach dem Auflaufen der Pflanzen (Kontaktwirkung)
Eine Anzahl Unkräuter und Kulturpflanzen, sowohl monokotyle wie dikotyle, wurden nach dem Auflaufen, im 4- bis 6-Blattstadium mit einer wässrigen Wirkstoffdispersion von 4 kg AS/ha gespritzt und dann bei 24° bis 26°C und 45-60 % relativer Luftfeuchtigkeit gehalten. 15 Tage nach der Behandlung wird der Versuch ausgewertet und nach dem gleichen Maßstab wie im pre-emergenten Versuch bewertet.
post-emergente Wirkung
Aufwandmenge: 4 kg Wirksubstanz/Hektar

| Verb.<br>Nr. | Avena | Setaria | Lolium | Solanum | Sinapis | Stellaria | Phaseolus |
|---|---|---|---|---|---|---|---|
| 4.31 | 9 | 6 | 5 | 7 | 2 | 3 | 5 |

**Beispiel 14:**

Wuchshemmung bei tropischen Bodendecker-Leguminosen (cover crops).
Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux

Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen) ohne dass dabei die Versuchspflanzen geschädigt wurden.

**Beispiel 15:**

Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines wirkstoff der Formel I bis zur guten Benetzung besprüht. Die Wirkstoffkonzentration beträgt bis zu 100 g AS/ha. Die Auswertung erfolgt ca. 5 wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten am Haupttrieb.

**Beispiel 16: Wuchshemmung bei Getreide**

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zur unbehandelten Kontrolle eine Verringerung des Neuzuwachses (60-90 % der Kontrolle), sowie teilweise eine Zunahme der Stengeldurchmesser auf.

**Beispiel 17: Wuchshemmung bei Gräsern**

In Kunststoffschalen mit Erde-Torf-Sand-Gemisch (6:3:1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchentlich bis auf 4 cm Hohe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

**Patentansprüche** für die Vertragsstaaten BE, CH, DE, FR, GB, IT, LI, NL, SE.

1. Phosphorhaltige N-Phenylsulfonyl-N'-triazinyl- und -pyrimidinylharnstoffe der Formel I

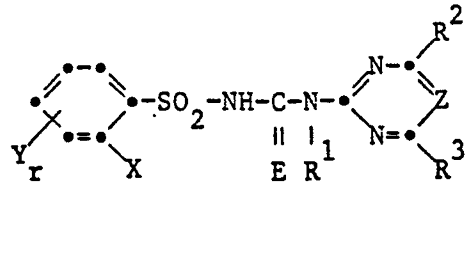 (I)

worin

X eine Gruppe

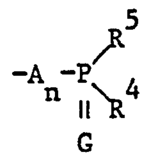 ,

Y Wasserstoff, Halogen, $C_1$-$C_5$-Alkyl, Trifluormethyl, $C_2$-$C_5$-Alkenyl, $C_2$-$C_5$-Alkinyl, Nitro, -COOR$^6$ oder -Q-R$^6$,
Z Stickstoff oder die Methingruppe,
E Sauerstoff oder Schwefel,
R$^1$ Wasserstoff oder $C_1$-$C_5$-Alkyl und
R$^2$ und R$^3$ unabhängig voneinander $C_1$-$C_3$-Alkyl, $C_1$-$C_3$-Alkoxy, $C_1$-$C_3$-Haloalkyl, $C_1$-$C_3$-Halogenalkoxy, Cyclopropyl, Amino, Methylamino oder Dimethylamino bedeuten, wobei
A für Sauerstoff, Schwefel, $C_1$-$C_5$-Alkylen, $C_2$-$C_5$-Alkenylen, $C_1$-$C_5$-Halogenalkylen, -NR$^7$ -$(CH_2)_m$-; -$CH_2$-NH-$(CH_2)_m$-; -$(CH_2)_m$-NR$^7$ -, -O-$(CH_2)_p$ -, -S-$(CH_2)_p$ -, -$(CH_2)_p$-O- oder -$(CH_2)_p$-S,
n für Null oder eins,
G für Sauerstoff oder Schwefel,
R$^4$ für $C_1$-$C_5$-Alkoxy, $C_1$-$C_5$-Halogenalkoxy, $C_1$-$C_5$-Alkylthio, $C_1$-$C_5$-Alkyl, $C_1$-$C_5$-Halogenalkyl, Phenyl oder Hydroxyl,
R$^5$ für Wasserstoff, $C_1$-$C_5$-Alkoxy, $C_1$-$C_5$-Halogenalkoxy, $C_1$-$C_5$-Alkylthio, $C_1$-$C_5$-Alkyl oder Hydroxyl,
R$^6$ für $C_1$-$C_5$-Alkyl, $C_1$-$C_5$-Halogenalkyl oder $C_2$-$C_6$-Alkoxyalkyl,
Q für Sauerstoff, Schwefel, -SO- oder -$SO_2$-,
R$^7$ für Wasserstoff, $C_1$-$C_5$-Alkyl, Phenyl, Benzyl oder durch $C_1$-$C_5$-Alkyl, Halogen oder Nitro substituiertes Phenyl,
m für eine Zahl von Null bis drei
p für eine Zahl von Null bis zwei und
r für eins oder zwei stehen,
sowie die Salze dieser Verbindungen.

2. Verbindungen gemäss Anspruch 1, dadurch gekennzeichnet, dass die Brücke $A_n$ eine direkte Bindung, $C_2$-$C_3$-Alkylen oder $C_2$-$C_3$-Alkenylen bedeutet.

3. Verbindungen gemäss Anspruch 1, dadurch gekennzeichnet, dass E Sauerstoff bedeutet.

4. Verbindungen gemäss Anspruch 1, dadurch gekennzeichnet, dass R$^1$ Wasserstoff bedeutet.

5. Verbindungen gemäss Anspruch 1, dadurch gekennzeichnet, dass die Reste R$^2$ und R$^3$ für Methyl oder Methoxy stehen.

6. Verbindungen gemäss Anspruch 1, dadurch gekennzeichnet, dass G Sauerstoff bedeutet.

7. Verbindungen gemäss Anspruch 1, dadurch gekennzeichnet, dass Y Wasserstoff bedeutet.

8. Verbindungen gemäss Anspruch 1, dadurch gekennzeichnet, dass die Brücke An eine direkte Bindung, $C_2$-$C_3$-Alkylen oder $C_2$-$C_3$-Alkenylen, G Sauerstoff und R$^4$ und R$^5$ unabhängig voneinander $C_1$-$C_5$-Alkyl oder $C_1$-$C_5$-Alkoxy bedeuten.

9. Verbindungen gemäss Anspruch 1, dadurch gekennzeichnet, dass E Sauerstoff, Y und R$^1$ Wasserstoff und R$^2$ und R$^3$ Methyl oder Methoxy bedeuten.

10. Verbindungen gemäss Anspruch 1, dadurch gekennzeichnet, dass E und G Sauerstoff, Y und R$^1$ Wasserstoff, R$^2$ und R$^3$ Methyl oder Methoxy, die Brücke $A_n$ eine direkte Bindung, $C_2$-$C_3$-Alkylen oder $C_2$-$C_3$-Alkenylen und R$^4$ und R$^5$ $C_1$-$C_5$-Alkyl oder $C_1$-$C_5$-Alkoxy bedeuten.

11. N-[2-(Diäthoxyphosphonyl)-phenylsulfonyl]-N'-(4,6-dimethoxy-pyrimidin-2-yl)-harnstoff gemäss Anspruch 1.

12. N-[2-(Di-n-butyloxyphosphonylvinyl)-phenylsulfonyl]-N'-(4-methoxy-6-methyl-pyrimidin-2-yl)-harnstoff gemäss Anspruch 1.

13. Verfahren zur Herstellung der Verbindungen der Formel I, Anspruch 1, dadurch gekennzeichnet, dass man ein Phenylsulfonamid der Formel II

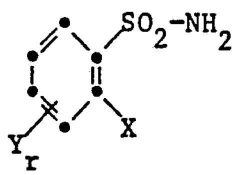 (II),

worin X, Y und r die unter Formel I gegebene Bedeutung haben, in Gegenwart einer Base mit einem N-Pyrimidinyl- oder -Triazinylcarbamat der Formel III

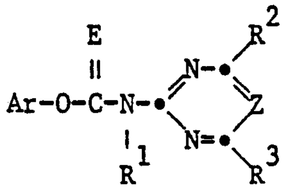

(III),

worin E, $R^1$ $R^2$ $R^3$ und Z die unter Formel I gegebene Bedeutung haben und Ar für Phenyl oder durch $C_1$-$C_5$-Alkyl, Halogen, $C_1$-$C_5$-Alkoxy oder Nitro substituiertes Phenyl steht, umsetzt und gegebenenfalls in ihre Salze überführt.

14. Verfahren zur Herstellung der Verbindungen der Formel I, Anspruch 1, dadurch gekennzeichnet, dass man ein Phenylsulfonylisocyanat oder -isothiocyanat der Formel IV

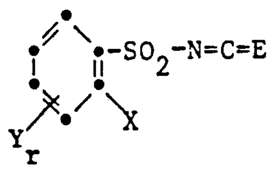

(IV),

worin

E, X, Y und r die unter Formel I gegebene Bedeutung haben, gegebenenfalls in Gegenwart einer Base, mit einem Amin der Formel V

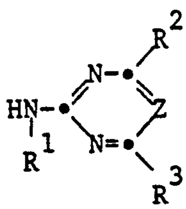

(V),

worin Z, $R^1$, $R^2$ und $R^3$ die unter Formel I gegebene Bedeutung haben, umsetzt und gegebenenfalls in ihre Salze überführt.

15. Verfahren zur Herstellung von Salzen der Formel I gemäss einem der Ansprüche 13 oder 14, dadurch gekennzeichnet, dass man einen Sulfonylharnstoff der Formel I mit einem Amin, einem Alkalimetalloder Erdalkalimetallhydroxid oder einer quaternären Ammoniumbase umsetzt.

16. Ein herbizides und den Pflanzenwuchs regulierendes Mittel, dadurch gekennzeichnet, dass es neben Träger- und/oder anderen Zuschlagstoffen als Wirkstoff mindestens einen N-Phenylsulfonyl-N'-triazinyl- oder -pyrimidinyl-harnstoff der Formel I, Anspruch 1, enthält.

17. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

18. Die Verwendung der N-Phenylsulfonyl-N'-triazinyl- oder -pyrimidinyl-harnstoffe der Formel I, Anspruch 1, oder sie enthaltender Mittel zur Regulierung des Pflanzenwachstums.

19. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

20. Phenylsulfonamide der Formel II

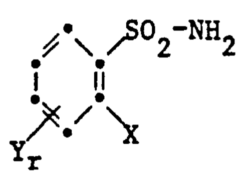

worin X, Y und r die unter Formel I im Anspruch 1 gegebene Bedeutung haben, unter Ausnahme von 0-(2-Sulfamoylphenyl)-0,0-dimethyl-phosphat.

**Patentansprüche** für den Vertragsstaat AT

1. Ein herbizides und den Pflanzenwuchs regulierendes Mittel, dadurch gekennzeichnet, dass es neben Träger- und/oder anderen Zuschlagstoffen als Wirkstoff mindestens einen phosphorhaltigen N-Phenylsulfonyl-N'-triazinyl- und -pyrimidinyl-harnstoff der Formel I

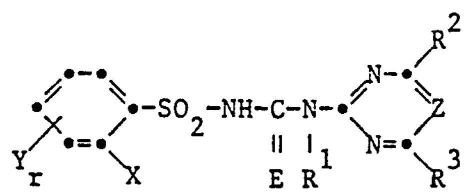

(I)

enthält, worin
X eine Gruppe

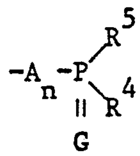

,

Y Wasserstoff, Halogen $C_1$-$C_5$-Alkyl, Trifluormethyl, $C_2$-$C_5$-Alkenyl, $C_2$-$C_5$-Alkinyl, Nitro, -$COOR^6$ oder -Q-$R^6$,
Z Stickstoff oder die Methingruppe,
E Sauerstoff oder Schwefel,
$R^1$ Wasserstoff oder $C_1$-$C_5$-Alkyl und
$R^2$ und $R^3$ unabhängig voneinander $C_1$-$C_3$-Alkyl, $C_1$-$C_3$-Alkoxy, $C_1$-$C_3$-Haloalkyl, $C_1$-$C_3$-Halogenalkoxy, Cyclopropyl, Amino, Methylamino oder Dimethylamino bedeuten, wobei
A für Sauerstoff, Schwefel, $C_1$-$C_5$-Alkylen, $C_2$-$C_5$-Alkenylen, $C_1$-$C_5$-Halogenalkylen, -$NR^7$-$(CH_2)_m$-; -$CH_2$-NH-$(CH_2)_m$-; -$(CH_2)_m$-$NR^7$-, -O-$(CH_2)_p$-, -S-$(CH_2)_p$-, -$(CH_2)_p$-O- oder -$(CH_2)_p$-S-,
n für Null oder eins,
G für Sauerstoff oder Schwefel,
$R^4$ für $C_1$-$C_5$-Alkoxy, $C_1$-$C_5$-Halogenalkoxy, $C_1$-$C_5$-Alkylthio, $C_1$-$C_5$-Alkyl, $C_1$-$C_5$-Halogenalkyl, Phenyl oder Hydroxyl,
$R^5$ für Wasserstoff, $C_1$-$C_5$-Alkoxy, $C_1$-$C_5$-Halogenalk oxy, $C_1$-$C_5$-Alkylthio, $C_1$-$C_5$-Alkyl oder Hydroxyl,
$R^6$ für $C_1$-$C_5$-Alkyl, $C_1$-$C_5$-Halogenalkyl oder $C_2$-$C_6$-Alkoxyalkyl,
Q für Sauerstoff, Schwefel, -SO- oder -$SO_2$-,
$R^7$ für Wasserstoff, $C_1$-$C_5$-Alkyl, Phenyl, Benzyl oder durch $C_1$-$C_5$-Alkyl, Halogen oder Nitro substituiertes Phenyl,
m für eine Zahl von Null bis drei
P für eine Zahl von Null bis zwei und
r für eins oder zwei stehen,
oder die Salze dieser Verbindungen.

2. Mittel gemäss Anspruch 1, dadurch gekennzeichnet, dass die Brücke $A_n$ eine direkte Bindung, $C_2$-$C_3$-Alkylen oder $C_2$-$C_3$-Alkenylen bedeutet.

3. Mittel gemäss Anspruch 1, dadurch gekennzeichnet, dass E Sauerstoff bedeutet.

4. Mittel gemäss Anspruch 1, dadurch gekennzeichnet, dass $R^1$ Wasserstoff bedeutet.

5. Mittel gemäss Anspruch 1, dadurch gekennzeichnet, dass die Reste $R^2$ und $R^3$ für Methyl oder Methoxy stehen.

6. Mittel gemäss Anspruch 1, dadurch gekennzeichnet, dass G Sauerstoff bedeutet.

7. Mittel gemäss Anspruch 1, dadurch gekennzeichnet, dass Y Wasserstoff bedeutet.

8. Mittel gemäss Anspruch 1, dadurch gekennzeichnet, dass die Brücke $A_n$ eine direkte Bindung, $C_2$-$C_3$-Alkylen oder $C_2$-$C_3$-Alkenylen, G Sauerstoff und $R^4$ und $R^5$ unabhängig voneinander $C_1$-$C_5$-Alkyl oder $C_1$-$C_5$-Alkoxy bedeuten.

9. Mittel gemäss Anspruch 1, dadurch gekennzeichnet, dass E Sauerstoff, Y und $R^1$ Wasserstoff und $R^2$ und $R^3$ Methyl oder Methoxy bedeuten.

10. Mittel gemäss Anspruch 1, dadurch gekennzeichnet, dass E und G Sauerstoff, Y und $R^1$ Wasserstoff, $R^2$ und $R^3$ Methyl oder Methoxy, die Brücke $A_n$ eine direkte Bindung, $C_2$-$C_3$-Akylen oder $C_2$-$C_3$-Alkenylen und $R^4$ und $R^5$ $C_1$-$C_5$-Alkyl oder $C_1$-$C_5$-alkoxy bedeuten.

11. Mittel gemäss Anspruch 1, dadurch gekennzeichnet, dass es als Wirkstoff N-[2-(Diäthoxyphosphonyl)-phenylsulfonyl]-N'-(4,6-dimethox y-pyrimidin-2-yl)-harnstoff oder
N-[2-(Di-n-butyloxyphosphonylvinyl)-phenylsulfonyl]-N'-(4-methoxy-6-methyl-pyrimidin-2-yl)-harnstoff

enthält.

12. Verfahren zur Herstellung der Verbindungen der Formel I, Anspruch 1, dadurch gekennzeichnet, dass man entweder

a) ein Phenylsulfonamid der Formel II

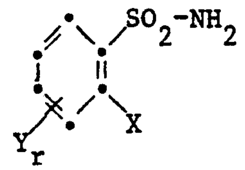

(II),

worin X, Y und r die unter Formel I gegebene Bedeutung haben, in Gegenwart einer Base mit einem N-Pyrimidinyl- oder -Triazinylcarbamat der Formel III

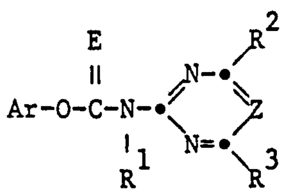

(III),

worin E, $R_1$, $R_2$, $R_3$ und Z die unter Formel I gegebene Bedeutung haben und Ar für Phenyl oder durch $C_1$-$C_5$-Alkyl, Halogen, $C_1$-$C_5$-Alkoxy oder Nitro substituiertes Phenyl steht, umsetzt oder

b) ein Phenylsulfonylisocyanat oder -isothiocyanat der Formel IV

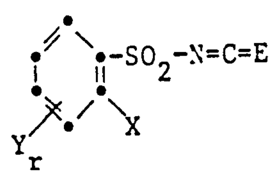

(IV),

worin

E, X, Y und r die unter Formel I gegebene Bedeutung haben, gegebenenfalls in Gegenwart einer Base, mit einem Amin der Formel V

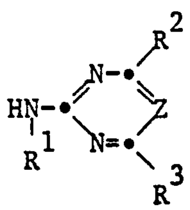

(V),

worin Z, $R^1$, $R^2$ und $R^3$ die unter Formel I gegebene Bedeutung haben, umsetzt und gegebenenfalls in ihre Salze überführt, indem man einen Sulfonylharnstoff der Formel I mit einem Amin, einem Alkalimetall oder Erdalkalimetallhydroxid oder einer quaternären Ammoniumbase umsetzt.

13 Die Verwendung der N-Phenylsulfonyl-N'-triazinyl- oder -pyrimidinyl-harnstoffe der Formel I, nach Anspruch 1, oder sie enthaltender Mittel zur Bekämpfung unerwünschten Pflanzenwachstums.

14. Die Verwendung der N-Phenylsulfonyl-N'-triazinyl- oder -pyrimidinyl-harnstoffe der Formel I, nach Anspruch 1, oder sie enthaltender Mittel zur Regulierung des Pflanzenwachstums.

15. Die Verwendung der N-Phenylsulfonyl-N'-triazinyl- oder -pyrimidinyl-harnstoffe der Formel I, nach Anspruch 1,oder sie enthaltender Mittel zur Wuchsregulierung von Kulturpflanzen zum Zwecke einer Ertragssteigerung.

**Claims** for the Contracting States: BE, CH, DE, FR, GB, IT, LI, NL, SE.

1. A phosphorus-containing N-phenylsulfonyl-N'-triazinylurea or N-phenylsulfonyl-N'-pyrimidinylurea of the formula

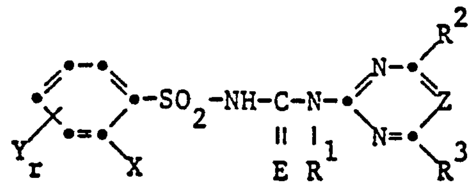

(I)

wherein
X is a group

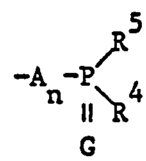

,

Y is hydrogen, halogen, $C_1$-$C_5$ alkyl, trifluoromethyl, $C_2$-$C_5$ alkenyl, $C_2$-$C_5$ alkynyl, nitro, $-COOR^6$ or $-Q-R^6$
Z is nitrogen or the methine group,
E is oxygen or sulfur,
$R^1$ is hydrogen or $C_1$-$C_5$ alkyl and,
$R^2$ and $R^3$ are each independently of the other $C_1$-$C_3$ alkyl, $C_1$-$C_3$ alkoxy, $C_1$-$C_3$ haloalkyl, $C_1$-$C_3$ haloalkoxy, cyclopropyl, amino, methylamine or dimethylamino, and
A is oxygen, sulfur, $C_1$-$C_5$ alkylene, $C_2$-$C_5$ alkenylene, $C_1$-$C_5$ haloalkylene or $-NR^7-(CH_2)m-$; $-CH_2-NH-(CH_2)_m-$; $-(CH_2)_m-NR^{-7}$ or $-O-(CH_2)_p-$, $-S-(CH_2)_p-$, $-(CH_2)_p-O-$ or $-(CH_2)_p-S-$,
n is zero or one,
G is oxygen or sulfur,
$R^4$ is $G_1$-$C_5$ alkoxy, $C_1$-$C_5$ haloalkoxy, $C_1$-$C_5$ alkylthio, $C_1$-$C_5$ alkyl, $C_1$-$C_5$ haloalkyl, phenyl or hydroxyl,
$R^5$ is hydrogen, $C_1$-$C_5$ alkoxy, $C_1$-$C_5$ haloalkoxy, $C_1$-$C_5$ alkylthio, $C_1$-$C_5$ alkyl or hydroxyl,
$R^6$ is $C_1$-$C_5$ alkyl, $C_1$-$C_5$ haloalkyl or $C_2$-$C_6$ alkoxyalkyl,
Q is oxygen, sulfur, -SO- or $-SO_2-$,
$R^7$ is hydrogen, $C_1$-$C_5$ alkyl, phenyl, benzyl, or phenyl which is substituted by $C_1$-$C_5$ alkyl, halogen or nitro,
m is a number from zero to three,
p is a number from zero to two and
r is one or two,
or a salt thereof.

2. A compound as claimed in claim 1, wherein the bridge $A_n$ is a direct bond, $C_2$-$C_3$ alkylene or $C_2$-$C_3$ alkenylene.

3. A compound as claimed in claim 1, wherein E is oxygen.

4. A compound as claimed in claim 1, wherein $R^1$ is hydrogen.

5. A compound as claimed in claim 1, wherein the substituents $R^2$ and $R^3$ are methyl or methoxy.

6. A compound as claimed in claim 1, wherein G is oxygen.

7. A compound as claimed in claim 1, wherein Y is hydrogen.

8. A compound as claimed in claim 1, wherein the bridge $A_n$ is a direct bond, $C_2$-$C_3$ alkylene or $C_2$-$C_3$ alkenylene, G is oxygen and $R^4$ and $R^5$ are each independently of the other $C_1$-$C_5$ alkyl or $C_1$-$C_5$ alkoxy.

9. A compound as claimed in claim 1, wherein E is oxygen, Y and $R^1$ are hydrogen and $R^2$ and $R^3$ are methyl or methoxy.

10. A compound as claimed in claim 1, wherein E and G are oxygen, Y and $R^1$ are hydrogen, $R^2$ and $R^3$ are methyl or methoxy, the bridge $A_n$ is a direct bond, $C_2$-$C_3$ alkylene or $C_2$-$C_3$ alkenylene and $R^4$ and R are $C_1$-$C_5$ alkyl or $C_1$-$C_5$ alkoxy.

11. N-[2-(Diethoxyphosphonyl)phenylsulfonyl]-N'-(4,6-dimethoxypyrimidin-2-yl)urea as claimed in claim 1.

12. N-[2-(Di-n-butyloxyphosphonylvinyl)phenylsulfonyl]-N'-(methoxy-6-methylpyrimidin-2-yl)urea as claimed in claim 1.

13. A process for the preparation of a compound of formula I according to claim 1, which comprises reacting a phenylsulfonamide of the formula II

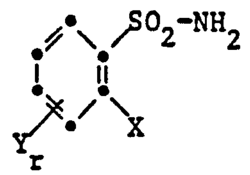

(II),

in which X, Y and r are as defined for formula I, in the presence of a base, with an N-pyrimidinylcarbamate or N-triazinylcarbamate of the formula III

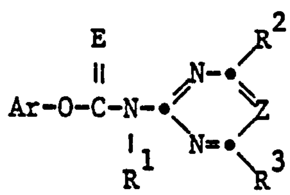

(III),

in which E, $R^1$, $R^2$, $R^3$ and Z are as defined for formula I and Ar is phenyl or phenyl which is substituted by $C_1$-$C_5$ alkyl, halogen, $C_1$-$C_5$ alkoxy or nitro, and, if desired, converting the resultant compound of formula I into a salt thereof.

14. A process for the preparation of a compound of formula I according to claim 1, which comprises reacting a phenylsulfonyl isocyanate or isothiocyanate of the formula IV

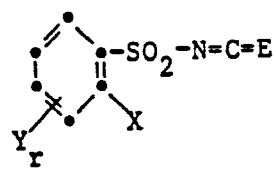

(IV),

in which E, X, Y and r are as defined for formula I, optionally in the presence of a base, with an amine of the formula V

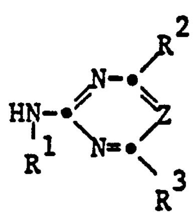

(V),

in which Z, $R^1$, $R^2$ and $R^3$ are as defined for formula I, and if desired, converting the resultant compound of formula I into a salt thereof.

15. A process for the preparation of an addition salt of the formula I as claimed in either of claims 13 or 14, which comprises reacting a sulfonylurea of the formula I with an amine, an alkali metal hydroxide or alkaline earth metal hydroxide or a quaternary ammonium base.

16. A herbicidal and plant growth-regulating composition which, in addition to carriers and/or other adjuvants, contains, as the active substance, at least one N-phenylsulfonyl-N'-triazinylurea or N-phenylsulfonyl-N'-Pyrimidinylurea of the formula I according to claim 1.

17. Use of an N-phenylsulfonyl-N'-triazinylurea or N-phenylsulfonyl-N'-pyrimidinylurea of formula I according to claim 1, or of a composition containing said compound, for controlling undesirable plant growth.

18. Use of an N-phenylsulfonyl-N'-triazinylurea or N-phenylsulfonyl-N'-pyrimidinylurea of formula I according to claim 1, or of a composition containing said compound, for regulating plant growth.

19. Use of an N-phenylsulfonyl-N'-triazinylurea or N-phenylsulfonyl-N'-pyrimidinylurea of formula I according to claim 1, or of a composition containing said compound, for regulating the growth of crop plants to achieve an increase in yield.

20. A phenylsulfonamide of the formula II

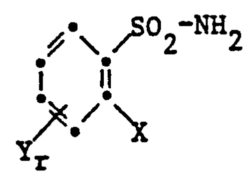

in which X, Y and r are as defined for formula I in claim 1, with the exception of 0-(2-sulfamoylphenyl)-0,0-dimethylphosphate.

**Claims** for the Contracting State: AT

1. A herbicidal and plant growth regulating composition which contains, in addition to carriers and/or other adjuvants, at least one phosphorus-containing N-phenylsulfonyl-N'-triazinylurea or N-phenylsulfonyl-N'-pyrimidinylurea of the formula

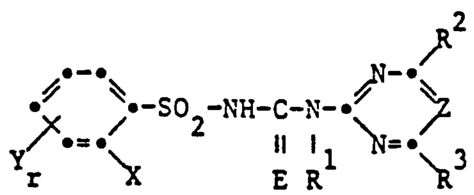

(I)

wherein X is a group

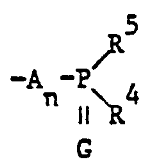

,

Y is hydrogen, halogen, $C_1$-$C_5$ alkyl, trifluoromethyl, $C_2$-$C_5$ alkenyl, $C_2$-$C_5$ alkynyl, nitro, -$COOR^6$ or -Q-$R^6$,
Z is nitrogen or the methine group,
E is oxygen or sulfur,
$R^1$ is hydrogen or $C_1$-$C_5$ alkyl and,
$R^2$ and $R^3$ are each independently of the other $C_1$-$C_3$ alkyl, $C_1$-$C_3$ alkoxy, $C_1$-$C_3$ haloalkyl, $C_1$-$C_3$ haloalkoxy, cyclopropyl, amino, methylamine or dimethylamino, and
A is oxygen, sulfur, $C_1$-$C_5$ alkylene, $C_2$-$C_5$ alkenylene, $C_1$-$C_5$ haloalkylene or $-NR^7-(CH_2)_m-$; $-CH_2-NH-(CH_2)_m-$; $-(CH_2)_m-NR^7-$ or $-O-(CH_2)_p$, $-S-(CH_2)_p-$, $-(CH_2)_p-O-$ or $-(CH_2)_p-S$,
n is zero or one,
C is oxygen or sulfur,
$R^4$ is $C_1$-$C_5$ alkoxy, $C_1$-$C_5$ haloalkoxy, $C_1$-$C_5$ alkylthio, $C_1$-$C_5$ alkyl, $C_1$-$C_5$ haloalkyl, phenyl or hydroxyl,
$R^5$ is hydrogen, $C_1$-$C_5$ alkoxy, $C_1$-$C_5$ haloalkoxy, $C_1$-$C_5$ alkylthio, $C_1$-$C_5$ alkyl or hydroxyl,
$R^6$ is $C_1$-$C_5$ alkyl $C_1$-$C_5$ haloalkyl or $C_2$-$C_6$ alkoxyalkyl,
Q is oxygen, sulfur, -SO- or -$SO_2$-,
$R^7$ is hydrogen $C_1$-$C_5$ alkyl, phenyl, benzyl, or phenyl which is substituted by $C_1$-$C_5$ alkyl, halogen or nitro,
m is a number from zero to three,
p is a number from zero to two and
r is one or two,
or a salt thereof.

2. A composition as claimed in claim 1, wherein the bridge $A_n$ is a direct bond, $C_2$-$C_3$ alkylene or $C_2$-$C_3$ alkenylene.
3. A composition as claimed in claim 1, wherein E is oxygen.
4. A composition as claimed in claim 1, wherein $R^1$ is hydrogen.
5. A composition as claimed in claim 1, wherein the radicals $R^2$ and $R^3$ are methyl or methoxy.
6. A composition as claimed in claim 1, wherein G is oxygen.
7. A composition as claimed in claim 1, wherein Y is hydrogen.

8. A composition as claimed in claim 1, wherein the bridge $A_n$ is a direct bond, $C_2$-$C_3$ alkylene or $C_2$-$C_3$ alkenylene, G is oxygen and $R^4$ and $R^5$ are each independently of the other $C_1$-$C_5$ alkyl or $C_1$-$C_5$ alkoxy.

9. A composition as claimed in claim 1, wherein E is oxygen, Y and $R^1$ are hydrogen and $R^2$ and $R^3$ are methyl or methoxy.

10. A composition as claimed in claim 1, wherein E and G are oxygen, Y and $R^1$ are hydrogen, $R^2$ and $R^3$ are methyl or methoxy, the bridge $A_n$ is a direct bond, $C_2$-$C_3$ alkylene or $C_2$-$C_3$ alkenylene and $R^4$ and $R^5$ are $C_1$-$C_5$ alkyl or $C_1$-$C_5$ alkoxy.

11. A composition as claimed in claim 1 which contains, as active ingredient N-[2-diethoxyphosphonyl)phenylfonyl]-N'- (4,6-dimethoxypyrimidin-2-yl)urea or N-[2-(di-n-butyloxyphosphonylvinyl)phenylsulfonyl]-N'-(4-methoxy-6-methylpyrimidin-2-yl)urea.

12. A process for the preparation of a compound of formula I according to claim 1, which comprises either

a) reacting a phenylsulfonamide of the formula II

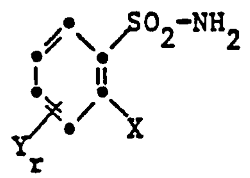
(II),

in which X, Y and r are as defined for formula I, in the presence of a base, with an N-pyrimidinylcarbamate or N-triazinylcarbamate of the formula III

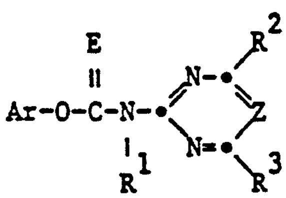
(III),

in which E, $R^1$, $R^2$, $R^3$ and Z are as defined for formula I and Ar is phenyl or phenyl which is substituted by $C_1$-$C_5$ alkyl, halogen, $C_1$-$C_5$ alkoxy or nitro, or

b) reacting a phenylsulfonyl isocyanate or isothiocyanate of the formula IV

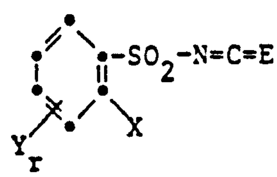
(IV),

in which E, X, Y and r are as defined under formula I, optionally in the presence of a base, with an amine of the formula V

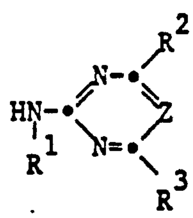
(V),

in which Z, $R^1$, $R^2$ and $R^3$ are as defined under formula I, and if desired, converting the compound of formula I so obtained into a salt thereof by reacting a sulfonylurea of the formula I with an amine, an alkali metal hydroxide or alkaline earth metal hydroxide or a quaternary ammonium base.

13. Use of an N-phenylsulfonyl-N'-triazinylurea or N-phenylsulfonyl-N'-pyrimidinylurea of the formula I according to claim 1, or of a composition containing said compound, for controlling undesirable plant growth.

14. Use of an N-phenylsulfonyl-N'-triazinylurea or N-phenylsulfonyl-N'-pyrimidinylurea of the formula I

according to claim 1, or of a composition containing said compound, for regulating plant growth.

15. Use of an N-phenylsulfonyl-N'-triazinylurea or N-phenylsulfonyl-N'-pyrimidinylurea of the formula I according to claim 1, or of a composition containing said compound, for regulating the growth of crop plants to achieve an increase in yield.

**Revendications** pour les Etats contractants: BE, CH, DE, FR, GB, IT, LI, NL, SE.

1. N-phénylsulfonyl-N'-triazinyl- et pyrimidinyl-urées contenant du phosphore de formule I

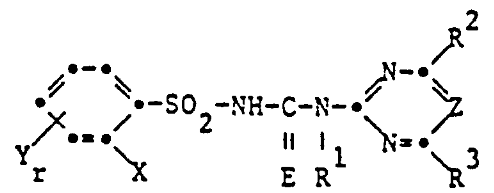 (I)

dans laquelle

X est un groupe $-A_n$

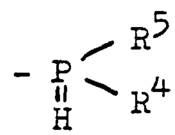

Y est un hydrogène, halogène, alkyle $C_1$-$C_5$-, trifluorométhyle, alcényle $C_2$-$C_5$-, alcynyle $C_2$-$C_5$-, nitro, $-COOR^6$ ou $-Q-R^6$

Z est l'azote ou le groupe méthine,

E est l'oxygène ou le soufre,

$R^1$ est un hydrogène ou alkyle $C_1$-$C_5$- et

$R^2$ et $R^3$ indépendamment l'un de l'autre représentent un alkyle $C_1$-$C_3$, alcoxy $C_1$-$C_3$, halogénoalkyle $C_1$-$C_3$, halogénoalcoxy $C_1$-$C_3$, cyclopropyle, amino, méthylamino ou diméthylamino, dans lesquels

A est l'oxygène, le soufre, un alkylène $C_1$-$C_5$, alcénylène $C_2$-$C_5$, halogénoalkylène $C_1$-$C_5$-, $NR^7$-$(CH_2)_m$-; $-CH_2$-$NH$-$(CH_2)_m$-; $-(CH_2)_m$-$NR^7$-, $-O$-$(CH_2)_p$-, $-S$-$(CH_2)_p$-, $-(CH_2)_p$-$O$- ou - $(CH_2)_p$-$S$-,

n est zéro ou un

G est l'oxygène ou le soufre

$R^4$ est un alcoxy $C_1$-$C_5$, halogénoalcoxy $C_1$-$C_5$-, alkylthio $C_1$-$C_5$-, alkyle $C_1$-$C_5$, halogénoalkyle $C_1$-$C_5$, phényle ou hydroxyle,

$R^5$ est un hydrogène, alcoxy $C_1$-$C_5$, halogénoalcoxy $C_1$-$C_5$, alkylthio $C_1$-$C_5$-, alkyle $C_1$-$C_5$ ou hydroxyle,

$R^6$ est un alkyle $C_1$-$C_5$, halogénoalkyle $C_1$-$C_5$ ou alcoxyalkyle $C_2$-$C_6$,

est l'oxygène le soufre, -SO- ou $-SO_2$

$R^7$ est un hydrogène, alkyle $C_1$-$C_5$-, phenyle, benzyle ou phényle substitué par alkyle $C_1$-$C_5$, halogène ou nitro,

m est un nombre de zéro à trois

p est un nombre de zéro à deux et

r est un ou deux

ainsi que les sels de ces composés.

2. Composés selon la revendication 1, caractérisés en ce que le pont $A_n$ est une liaison directe, un alkylène $C_2$-$C_3$ ou un alcénylène $C_2$-$C_3$.

3. Composés selon la revendication 1, caractérisés en ce que E est l'oxygène.

4. Composés selon la revendication 1, caractérisés en ce que $R^1$ est l'hydrogène.

5. Composés selon la revendication 1, caractérisés en ce que les restes $R^2$ et $R^3$ représentent un méthyle ou méthoxy.

6. Composés selon la revendication 1, caractérisés en ce que G est l'oxygène.

7. Composés selon la revendication 1, caractérisés en ce cue Y est l'hydrogène.

8. Composés selon la revendication 1, caractérisés en ce que le pont $A_n$ est une liaison directe, un alkylène $C_2$-$C_3$ ou un alcénylène $C_2$-$C_3$, G l'oxygène et $R^4$ et $R^5$ indépendanment l'un de l'autre représentent un alkyle $C_1$-$C_5$ ou alcoxy $C_1$-$C_5$.

9. Composés selon la revendication 1, caractérisés en ce que E est l'oxygène, Y et $R^1$ l'hydrogène et $R^2$ et $R^3$ représentent un méthyle ou méthoxy.

10. Composés selon la revendication 1, caractérisés en ce que E et G sont l'oxygène, Y et $R^1$ l'hydrogène, $R^2$ et $R^3$ un méthyle ou méthoxy, le pont $A_n$ une liaison directe, un alkylène $C_2$-$C_3$ ou alcénylène $C_2$-$C_5$ et $R^4$ et $R^5$ representent un alkyle $C_1$-$C_5$ ou alcoxy $C_1$-$C_5$.

11. N-[2-(diéthoxyphosphonyl)-phénylsulfonyl]-N'-(4,6-diméthoxy-pyrimidine-2-yl)-urée selon la revendication 1.

12. N-[2-(di-n-butyloxyphosphonylvinyl)-phénylsulfonyl]-N'-(4-méthoxy-6-méthyl-pyrimidine-2-yl)-urée selon la revendication 1.

13. Procédé de préparation des composés de formule I, revendication 1 caractérisé en ce qu'on fait réagir un phénylsulfonamide de formule II

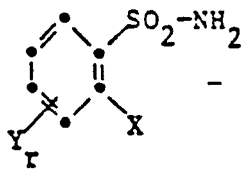

(II),

dans laquelle X, Y et r ont la signification indiquée pour la formule I, en présence d'une base avec un N-pyrimidinyl- ou triazinylcarbamate de formule III

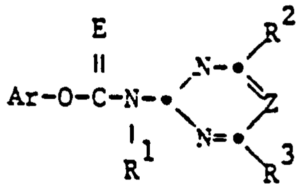

(III),

dans laquelle E, $R^1$, $R^2$, $R^3$ et Z ont la signification donnée pour la formule I et Ar est un phényle ou un phényle substitué par un alkyle $C_1$-$C_5$-, halogène, alcoxy $C_1$-$C_5$- ou nitro, et les transforme en leurs sels le cas échéant.

14. Procédé de préparation de composés de formule I, revendication 1, caractérisé en ce qu'on fait réagir un phénylsulfonylisocyanate ou isothiocyanate de formule IV

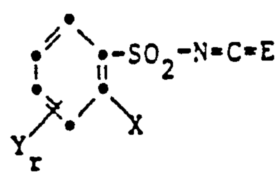

(IV),

dans laquelle E, X, Y et r ont la signification indiquée pour la formule I, le cas échéant en présence d'une base avec une amine de formule V

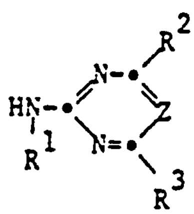

(V),

dans laquelle Z, $R^1$, $R^2$, et $R^3$ ont la signification donnée pour la formule I et les transforme en leurs sels le cas échéant.

15. Procédé de préparation de sels de formule I selon l'une des revendications 13 ou 14 caractérisé en ce qu'on fait réagir une sulfonylurée de formule I avec une amine, un hydroxyde de métal alcalin ou alcalino-terreux ou une base d'ammonium quaternaire.

16. Moyen herbicide et de régulation de croissance végétale, caractérisé en ce qu'outre les véhicules et/ou autres adjuvants il contient comme principe actif au moins une N-phénylsulfonyl-N-triazinyl- ou -pyrimidinyl-urée de formule I, revendication 1.

17. Utilisation de N-phénylaulfonyl-N'-triazinyl- ou -pyrimidinyl-urée de formule I, revendication 1, ou des moyens les contenant pour combattre la croissance végétale indésirable.

18. Utilisation de N-phénylsulfonyl-N'-triazinyl- ou -pyrimidinyl-urée de formule I, revendication 1, ou des moyens les contenant pour réguler la croissance végétale.

19. Utilisation de N-phénylsulfonyl-N'-triazinyl- ou -pyrimidinyl-urées de formule I, revendication 1, ou des moyens les contenant pour la régulation de la croissance de plantes cultivées dans le but d'augmenter le rendement.

20. Phénylsulfonamides de formule II

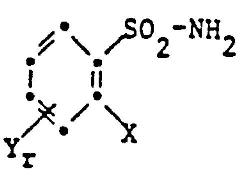

dans laquelle X, Y et r ont la signification donnée pour la formule I dans la revendication 1, à l'exception de 1'0-(2-sulfamoylphényl)-0,0-diméthyl-phosphate.

**Revendications** pour l'Etat contractant: AT

1. Moyen herbicide et régulateur de la croissance végétale, caractérisé en ce qu'outre les véhicules et/ou autres adjuvants il contient au moins une N-phénylsulfonyl-N'-triazinyl ou pyrimidinyl-urée de formule I

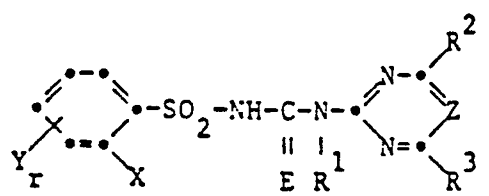

(I)

dans laquelle

X est un groupe $-A_n$

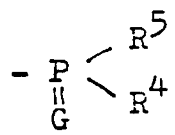

Y est un hydrogène, halogène, alkyle $C_1$-$C_5$, trifluorométhyle, alcényle $C_2$-$C_5$, alcynyle $C_2$-$C_5$, nitro, $-COOR^6$ ou $-Q-R^6$,

Z est l'azote ou le groupe méthine,

E est l'oxygène ou le soufre,

$R^1$ est un hydrogène ou alkyle $C_1$-$C_5$ et

$R^2$ et $R^3$ indépendamment l'un de l'autre représentent un alkyle $C_1$-$C_3$, alcoxy $C_1$-$C_3$, halogénoalkyle $C_1$-$C_3$, halogénoalcoxy $C_1$-$C_3$, cyclopropyle, amino, méthylamino ou diméthylamino, dans lesquels

A est l'oxygène, le soufre, un alkylène $C_1$-$C_5$, alcénylène $C_2$-$C_5$, halogénoalkylène $C_1$-$C_5$-, $-NR^7-(CH_2)_m-$; $-CH_2-NH-(CH_2)_m-$; $-(CH_2)_m-NR^7-$, $-O-(CH_2)_p$, $-S-(CH_2)_p-$, $(CH_2)$ -O- ou $-(CH_2)_p-S-$,

n est zéro ou un

G est l'oxygène ou le soufre

$R^4$ est un alcoxy $C_1$-$C_5$, halogénoalcoxy $C_1$-$C_5$-, alkylthio $C_1$-$C_5$-, alkyle $C_1$-$C_5$, halogénoalkyle $C_1$-$C_5$, phényle ou hydroxyle,

$R^5$ est un hydrogène, alcoxy $C_1$-$C_5$, halogénoalcoxy $C_1$-$C_5$, alkylthio $C_1$-$C_5$- alkyle $C_1$-$C_5$ ou hydroxyle,

$R^6$ est un alkyle $C_1$-$C_5$ halogénoalkyle $C_1$-$C_5$ ou alcoxyalkyle $C_2$-$C_6$,

Q est l'oxygène, le soufre, -SO- ou $-SO_2$

$R^7$ est un hydrogène, alkyle $C_1$-$C_5$-, phényle, benzyle, ou phényle substitué par alkyle $C_1$-$C_5$, halogène ou nitro,

m est un nombre de zéro à trois

p est un nombre de zéro à deux et

r est un ou deux

ainsi que les sels de ces composés.

2. Moyen selon la revendication 1, caractérisé en ce que le pont $A_n$ est une liaison directe, un alkylène $C_2$-$C_3$ ou un alcénylène $C_2$-$C_3$.

3. Moyen selon la revendication 1, caractérisé en ce que E est l'oxygène.

4. Moyen selon la revendication 1, caractérisé en ce que $R^1$ est l'hydrogène.

5. Moyen selon la revendication 1, caractérisé en ce que les restes $R^2$ et $R^3$ représentent un méthyle ou méthoxy.

6. Moyen selon la revendication 1, caractérisé en ce que G est l'oxygène.

7. Moyen selon la revendication 1, caractérisé en ce que Y est l'hydrogène.

8. Moyen selon la revendication 1, caractérisé en ce que le pont $A_n$ est une liaison directe, un alkylène $C_2$-$C_3$ ou un alcénylène $C_2$-$C_3$, G l'oxygène et $R^4$ et $R^5$ indépendamment l'un de l'autre représentent un alkyle $C_1$-$C_5$ ou alcoxy $C_1$-$C_5$.

9. Moyen selon la revendication 1, caractérisé en ce que E est l'oxygène, Y et $R^1$ l'hydrogène et $R^2$ et $R^3$ représentent un méthyle ou méthoxy.

10. Moyen selon la revendication 1, caractérisé en ce que E et G sont l'oxygène, Y et $R^1$ l'hydrogène, $R^2$ et $R^3$ un méthyle ou méthoxy, le pont $A_n$ une liaison directe, un alkylène $C_2$-$C_3$ ou alcénylène $C_2$-$C_3$ et $R^4$ et $R^5$ représentent un alkyle $C_1$-$C_5$ ou alcoxy $C_1$-$C_5$.

11. Moyen selon la revendication 1, caractérisé en ce qu'il contient comme principe actif de la N-[2-(diéthoxyphosphonyl)-phénylsulfonyl]-N'-(4,6-diméthoxy-pyrimidine-2-yl)-urée ou de la N-[2-(di-n-butyloxyphosphonylvinyl)-phénylsulfonyl]-N'-(4-méthoxy-6-méthyl-pyrimidine-2-yl)-urée.

12. Procédé de préparation des composés de la formule I, revendication 1, caractérisé en ce que soit

a) on fait réagir un phénylsulfonamide de formule II

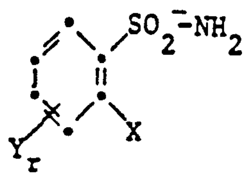

(II),

dans laquelle X, Y et r ont la signification indiquée pour la formule I, en présence d'une base avec un N-pyrimidinyl- ou triazinylcarbamate de formule III

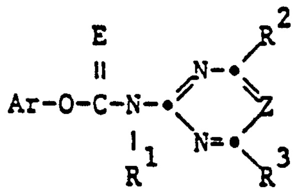

(III),

dans laquelle E, $R^1$, $R^2$, $R^3$ et Z ont la signification donnée pour la formule I et Ar est un phényle ou un phényle substitué par un alkyle $C_1$-$C_5$-, halogène, alcoxy $C_1$-$C_5$ ou nitro, soit

b) on fait réagir un phénylsulfonylisocyanate ou isothiocyanate de formule IV

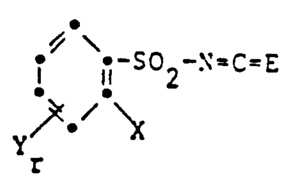

(IV),

dans laquelle E, X, Y et r ont la signification indiquée pour la formule I, le cas échéant en présente d'une base avec une amine de formule V

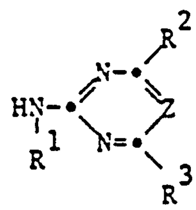

(V),

dans laquelle $R^1$, $R^2$ et $R^3$ ont la signification donnée pour la formule I, et transforme en leurs sels le cas échéant, en faisant réagir une sulfonylurée de formule I avec une amine, un hydroxyde de métal alcalin ou alcalino-terreux ou une base d'ammonium quaternaire.

13. Utilisation de N-phénylsulfonyl-N'-triazinyl- ou pyrimidinyl-urées de formule I, selon la revendication 1, ou des moyens les contenant pour combattre la croissance végétale indésirable.

14. Utilisation de N-phénylsulfonyl-N'-triazinyl- ou -pyrimidinylurées de formule I, selon la revendication 1, ou des moyens les contenant pour réguler la croissance végétale.

15. Utilisation de N-phénylsulfonyl-N'-triazinyl- ou -pyrimidinyl-urées de formule I, selon la revendication 1, ou des moyens les contenant pour la régulation de croiasance de plantes cultivées dans le but d'augmenter le rendement.